(19) Europäisches Patentamt
European Patent Office
Office européen des brevets

(11) Veröffentlichungsnummer: **0 431 424 A2**

# (12) EUROPÄISCHE PATENTANMELDUNG

(21) Anmeldenummer: **90122434.5**

(22) Anmeldetag: **24.11.90**

(51) Int. Cl.5: **C07D 401/04**, A01N 43/54

(30) Priorität: **07.12.89 DE 3940476**

(43) Veröffentlichungstag der Anmeldung:
**12.06.91 Patentblatt 91/24**

(84) Benannte Vertragsstaaten:
**BE CH DE DK ES FR GB IT LI NL SE**

(71) Anmelder: **BAYER AG**

**W-5090 Leverkusen 1 Bayerwerk(DE)**

(72) Erfinder: **Heinemann, Ulrich, Dr.**
**Feldstrasse 18**
**W-5653 Leichlingen 1(DE)**
Erfinder: **Klausener, Alexander, Dr.**
**Dahlerdyk 173**
**W-4150 Krefeld 1(DE)**
Erfinder: **Berg, Dieter, Dr.**
**Gellertweg 27**
**W-5600 Wuppertal 1(DE)**
Erfinder: **Dutzmann, Stefan, Dr.**
**Leinenweberweg 33**
**W-4000 Düsseldorf 13(DE)**
Erfinder: **Brandes, Wilhelm, Dr.**
**Eichendorffstrasse 3**
**W-5653 Leichlingen(DE)**

## (54) Pyridinylpyrimidin-Derivate.

(57) Beschrieben werden neue Pyridinylpyrimidin-Derivate der Formel (I)

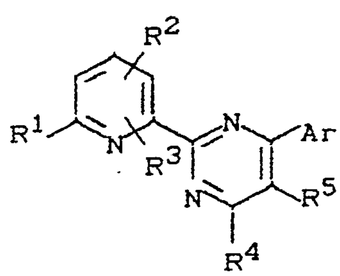

(I)

in welcher
$R^1$, $R^2$, $R^3$, $R^4$, $R^5$ und Ar die in der Beschreibung angegebene Bedeutung haben sowie mehrere Verfahren zu deren Herstellung.

Verwendet werden die neuen Verbindungen der Formel (I) zur Bekämpfung von Schädlingen.

EP 0 431 424 A2

## PYRIDINYLPYRIMIDIN-DERIVATE

Die Erfindung betrifft neue Pyridinylpyrimidin-Derivate, mehrere Verfahren zu ihrer Herstellung und ihre Verwendung als Schädlingsbekämpfungsmittel, insbesondere als Fungizide.

Es ist bekannt, daß bestimmte Pyridinylpyrimidin-Derivate wie beispielsweise das 2-(6-Methyl-2-pyridinyl)-4-o-tolylpyrimidin, das 2-(6-Methyl-2-pyridinyl)-4-hydroxy-6-phenylpyrimidin (vgl. EP 0 270 362) und das 2-(6-Phenyl-2-pyridinyl)-4-chlor-6-methylpyrimidin (vgl. EP 0 259 139) fungizide Eigenschaften besitzen.

Die fungizide Wirksamkeit dieser vorbekannten Verbindungen ist jedoch nicht in allen Anwendungsgebieten völlig zufriedenstellend.

Weiterhin sind das 4-Phenyl-2-(2-pyridyl)-pyrimidin und das 4-Chlor-6-phenyl-2-(2-pyridyl)-pyrimidin (vgl. J. Org. Chem. 32 (5), 1591-6 (1967)) bekannt. Über die physiologische Wirksamkeit dieser Verbindungen wird jedoch nichts berichtet.

Es wurden neue Pyridinylpyrimidin-Derivate der allgemeinen Formel (I),

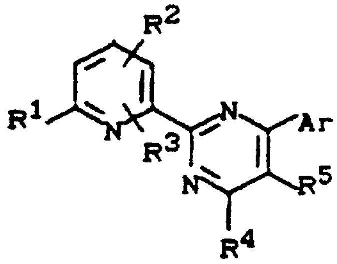

(I)

in welcher

| | |
|---|---|
| $R^1$ | für Wasserstoff, Halogen, Alkoxy, Alkylthio, Halogenalkyl, Amino oder Dialkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; |
| | in welcher weiterhin |
| $R^1$ | für jeweils gegebenenfalls substituiertes Aryloxy, Arylthio, Aralkyloxy oder Aralkylthio steht, |
| $R^2$ und $R^3$ | unabhängig voneinander, gleich oder verschieden jeweils für Wasserstoff, Halogen, Alkyl, Halogenalkyl, Alkoxy, Alkylthio oder Alkoxycarbonyl stehen, |
| | oder |
| $R^2$ und $R^3$ | gemeinsam für eine Alkylkette mit 3 bis 6 Kohlenstoffatomen stehen, die über die Ringpositionen 3 und 4 oder 4 und 5 verbunden sind, |
| $R^4$ | für Wasserstoff, Halogen, Alkyl, Halogenalkyl, Hydroxy, Alkoxy, Mercapto, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; oder |
| $R^4$ | für Alkenyloxy oder Alkinyloxy steht, |
| $R^5$ | für Wasserstoff oder Alkyl steht und |
| Ar | für unsubstituiertes oder substituiertes Aryl steht, |

gefunden, ausgenommen die Verbindungen 4-Phenyl-2-(2-pyridyl)-pyrimidin und 4-Chlor-6-phenyl-2-(2-pyridyl)-pyrimidin (bekannt aus J Org. Chem. 32 (5), 1591-6 (1967)).

Weiterhin wurde gefunden, daß man die neuen Pyridinylpyrimidin-Derivate der Formel (I)

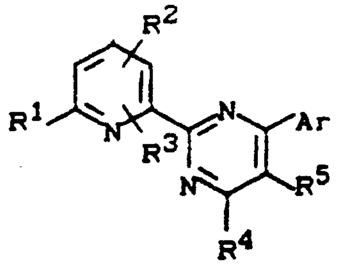

(I)

in welcher

$R^1$ für Wasserstoff, Halogen, Alkoxy, Alkylthio, Halogenalkyl, Amino oder Dialkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist;
in welcher weiterhin

$R^1$ für jeweils gegebenenfalls substituiertes Aryloxy, Arylthio, Aralkyloxy oder Aralkylthio steht,

$R^2$ und $R^3$ unabhängig voneinander, gleich oder verschieden jeweils für Wasserstoff, Halogen, Alkyl, Halogenalkyl, Alkoxy, Alkylthio oder Alkoxycarbonyl stehen,
oder

$R^2$ und $R^3$ gemeinsam für eine Alkylkette mit 3 bis 6 Kohlenstoffatomen stehen, die über die Ringpositionen 3 und 4 oder 4 und 5 verbunden sind,

$R^4$ für Wasserstoff, Halogen, Alkyl, Halogenalkyl, Hydroxy, Alkoxy, Mercapto, Alkylthio, Amino, (Di)alkylamino, steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist, oder

$R^4$ für Alkenyloxy oder Alkinyloxy steht,

$R^5$ für Wasserstoff oder Alkyl steht und

Ar für unsubstituiertes oder substituiertes Aryl steht,

nach einem der im folgenden beschriebenen Verfahren erhält.

A) Man erhält Pyridinylpyrimidin-Derivate der Formel (Ia)

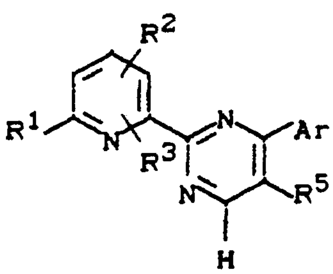

(Ia)

in welcher

$R^1$, $R^2$, $R^3$, $R^5$ und Ar die oben angegebene Bedeutung haben,

wenn man halogensubstituierte Pyridinylpyrimidin-Derivate der Formel (II)

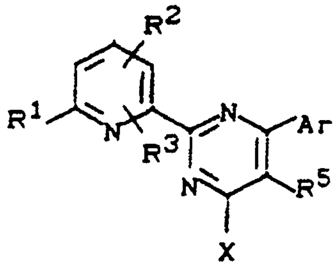

(II)

in welcher
$R^1$, $R^2$, $R^3$, $R^5$ und Ar die oben angegebene Bedeutung haben, und

X für Halogen steht,

gegebenenfalls in Gegenwart eines Lösungsmittels, gegebenenfalls in Gegenwart einer Base, in Gegenwart eines Katalysators und unter Wasserstoffdruck enthalogeniert,

oder

B) man erhält Pyridinylpyrimidin-Derivate der Formel (Ib)

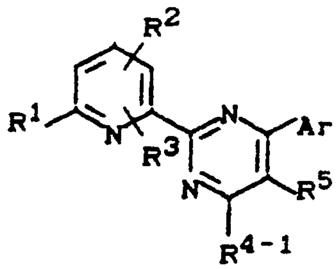

(Ib)

in welcher
$R^1$, $R^2$, $R^3$, $R^5$ und Ar die oben angegebene Bedeutung haben, und

$R^{4-1}$ für Mercapto, Alkoxy, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist oder für Alkenyloxy oder Alkinyloxy steht,

wenn man halogensubstituierte Pyridinylpyrimidin-Derivate der Formel (II)

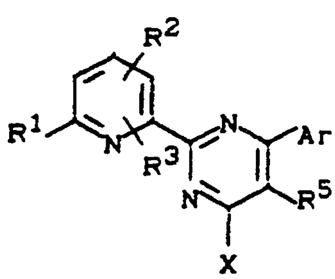

(II)

in welcher
$R^1$, $R^2$, $R^3$, $R^5$ und Ar die oben angegebene Bedeutung haben und

X für Halogen steht,

mit Verbindungen der Formel (III)

$$R^{4-1}-M \qquad (III)$$

in welcher

$R^{4-1}$ die oben angegebene Bedeutung hat und

M für Wasserstoff oder für ein Alkalimetallkation steht,

gegebenenfalls in Gegenwart eines Verdünnungsmittels und gegebenenfalls in Gegenwart einer Base umsetzt,

oder

C) man erhält Pyridinylpyrimidin-Derivate der Formel (Ic)

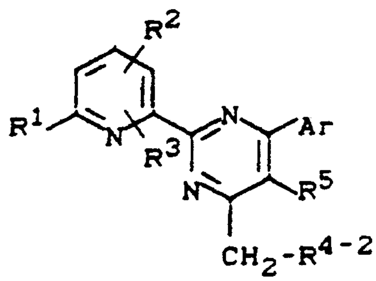

(Ic)

in welcher

$R^1$, $R^2$, $R^3$, $R^5$ und Ar die oben angegebene Bedeutung haben und

$R^{4-2}$ für Wasserstoff oder Alkyl steht,

wenn man halogensubstituierte Pyridinylpyrimidin-Derivate der Formel (II)

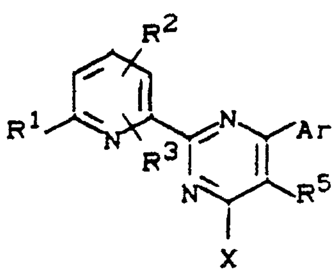

(II)

in welcher

$R^1$, $R^2$, $R^3$, $R^5$ und Ar die oben angegebene Bedeutung haben und

X für Halogen steht,

mit Diester-Derivaten der Formel (IV)

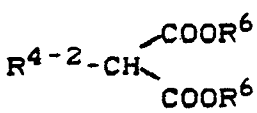

(IV)

in welcher

$R^{4-2}$ die oben angegebene Bedeutung hat und

$R^6$ für Methyl oder Ethyl steht,

gegebenenfalls in Gegenwart eines Verdünnungsmittels und gegebenenfalls in Gegenwart einer Base umsetzt und die so erhaltenen substituierten Pyridinylpyrimidine gegebenenfalls in Gegenwart eines Verdünnungsmittels und in Gegenwart einer Base hydrolysiert und anschließend in Gegenwart einer

Säure decarboxyliert;
oder
D) man erhält die Pyridinylpyrimidin-Derivate der Formel (Id)

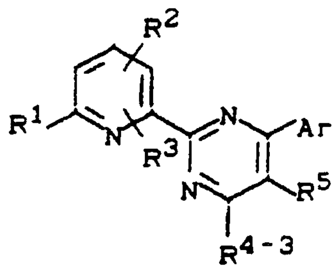

(Id)

in welcher
$R^1$, $R^2$, $R^3$, $R^5$ und Ar die oben angegebene Bedeutung haben und
$R^{4-3}$ für Wasserstoff, Alkyl oder Halogenalkyl steht,
wenn man Picolinamidin-Derivate der Formel (V)

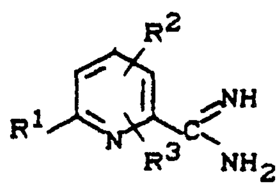

(V)

in welcher
$R^1$, $R^2$ und $R^3$ die oben angegebene Bedeutung haben, oder deren Säureadditionssalze
α) mit Arylketonen der Formel (VI)

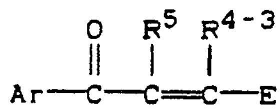

(VI)

in welcher
$R^5$ und Ar die oben angegebene Bedeutung haben,
$R^{4-3}$ für Wasserstoff, Alkyl oder Halogenalkyl steht und
E für eine Abgangsgruppe steht,
gegebenenfalls in Gegenwart eines Verdünnungsmittels und gegebenenfalls in Gegenwart einer Base cyclisiert,
oder
ß) mit Acetalen der Formel (VII)

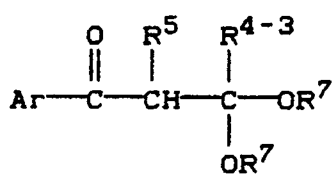

(VII)

in welcher

$R^5$ und Ar die oben angegebene Bedeutung haben,
$R^{4-3}$ für Wasserstoff, Alkyl oder Halogenalkyl steht und
$R^7$ für Alkyl mit 1 bis 4 Kohlenstoffatomen steht,

gegebenenfalls in Gegenwart eines Verdünnungsmittels und gegebenenfalls in Gegenwart einer Base umsetzt,

γ) mit 1,3-Dioxo-Derivaten der Formel (VIII)

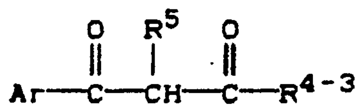
(VIII)

in welcher
$R^5$ und Ar die oben angegebene Bedeutung haben
und
$R^{4-3}$ für Wasserstoff, Alkyl oder Halogenalkyl steht,

gegebenenfalls in Gegenwart eines Verdünnungsmittels und in Gegenwart einer Base umsetzt,
oder

δ) mit Diacetalen der Formel (IX)

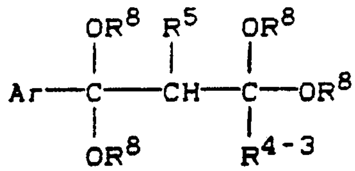
(IX)

in welcher
Ar und $R^5$ die oben angegebene Bedeutung haben,
$R^{4-3}$ für Wasserstoff, Alkyl oder Halogenalkyl steht und
$R^8$ für Alkyl mit 1 bis 4 Kohlenstoffatomen steht,

gegebenenfalls in Gegenwart eines Verdünnungsmittels umsetzt.

Schließlich wurde gefunden, daß die neuen Pyridinylpyrimidin-Derivate der Formel (I) eine gute Wirkung gegen Pflanzenkrankheiten zeigen.

Überraschenderweise zeigen die erfindungsgemäßen Pyridinylpyrimidin-Derivate der allgemeinen Formel (I) erheblich höhere fungizide Wirksamkeit als die aus dem Stand der Technik bekannten Pyridinylpyrimidin-Derivate wie beispielsweise das 2-(6-Methyl-2-pyridinyl)-4-hydroxy-6-phenylpyrimidin, das 2-(6-Methyl-2-pyridinyl)-4-o-tolylpyrimidin oder das 2-(6-Phenyl-2-pyridinyl)-4-chlor-6-methylpyrimidin, welche chemisch und wirkungsmäßig naheliegende Verbindungen sind.

Bevorzugte Substituenten bzw. Bereiche der in den oben und nachstehend erwähnten Formeln aufgeführten Reste werden im folgenden erläutert:

Für den Begriff Alkyl in den Definitionen von $R^1$, $R^2$, $R^3$, $R^4$ und $R^5$ in den allgemeinen Formeln, steht geradkettiges oder verzweigtes Alkyl mit vorzugsweise 1 bis 6, besonders bevorzugt 1 bis 4 und insbesondere 1 oder 2 Kohlenstoffatomen. Beispielhaft seien Methyl, Ethyl, n-Propyl, i-Propyl, n-Butyl, sec-Butyl, i-Butyl, t-Butyl, n-Pentyl, i-Pentyl und t-Pentyl genannt.

Für den Begriff Alkenyloxy in der Definition von $R^4$ in den allgemeinen Formeln steht geradkettiges oder verzweigtes Alkenyloxy mit vorzugsweise 2 bis 6, besonders bevorzugt 2 bis 4 und ganz besonders bevorzugt 2 oder 3 Kohlenstoffatomen. Beispielhaft seien Vinyloxy, Allyloxy, 2-Propenyloxy, 1-Butenyloxy, 2-Butenyloxy, 3-Butenyloxy und 1-Methallyloxy genannt.

Für den Begriff Alkinyloxy in der Definition von $R^4$ in den allgemeinen Formeln steht geradkettiges oder verzweigtes Alkinyloxy mit vorzugsweise 2 bis 6, besonders bevorzugt 2 bis 4 und ganz besonders bevorzugt 2 oder 3 Kohlenstoffatomen. Beispielhaft seien genannt 2-Propinyloxy, 1-Butinyloxy, 2-Butinyloxy und 3-Butinyloxy.

Der Begriff (Di)alkylamino in den Definitionen von $R^1$ und $R^4$ in den allgemeinen Formeln steht für eine Aminogruppe mit 1 oder 2 Alkylgruppen, welche jeweils geradkettig oder verzweigt, gleich oder verschieden

sein können und vorzugsweise jeweils 1 bis 6, insbesondere 1 bis 4 Kohlenstoffatome enthalten, wobei Methyl, Ethyl, n-und i-Propyl genannt seien. Beispielhaft seien Methylamino, Dimethylamino, Ethylamino, Diethylamino, n-Propylamino, Di-n-propylamino, i-Propylamino und Di-i-propylamino aufgeführt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

Unter dem Begriff unsubstituiertes oder substituiertes Aryl in der Definition von Ar in den allgemeinen Formeln ist Aryl mit vorzugsweise 6 bis 10 Kohlenstoffatomen im Arylteil zu verstehen. Beispielhaft und vorzugsweise seien unsubstituiertes oder substituiertes Phenyl oder Naphthyl, insbesondere Phenyl genannt.

Für den Begriff Halogenalkyl steht in den Definitionen von $R^1$, $R^2$, $R^3$ und $R^4$ in den allgemeinen Formeln vorzugsweise geradkettiges oder verzweigtes Alkyl mit je 1 bis 6 Kohlenstoffatomen, besonders bevorzugt mit 1 bis 4 Kohlenstoffatomen und jeweils 1 bis 13, vorzugsweise 1 bis 6, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlordifluor-methyl, Trifluorchlorethyl, Chlorbutyl, Fluorbutyl.

Unter dem Begriff Alkoxy in den Definitionen von $R^1$, $R^2$, $R^3$ und $R^4$ in den allgemeinen Formeln ist geradkettiges oder verzweigtes Alkoxy mit vorzugsweise 1 bis 6, insbesondere 1 bis 4 Kohlenstoffatomen zu verstehen. Beispielhaft seien Methoxy, Ethoxy, Propoxy, Butoxy sowie ihre Isomeren, i-Propoxy, i-, s- und t-Butoxy genannt.

Unter dem Begriff Alkylthio in den Definitionen von $R^1$, $R^2$, $R^3$ und $R^4$ in den allgemeinen Formeln ist geradkettiges oder verzweigtes Alkylthio mit vorzugsweise 1 bis 6 Kohlenstoffatomen, insbesondere 1 bis 4 Kohlenstoffatome zu verstehen. Beispielhaft seien Methylthio, Ethylthio, Propylthio, Butylthio, Pentylthio sowie ihre Isomeren, wie z.B. i-Propylthio, i-, s- und t-Butylthio genannt. Besonders bevorzugt sind Methylthio, Ethylthio, Tetrafluorethylthio, Trichlorethylthio, Chlordifluormethylthio und Trifluorchlorethylthio.

Unter dem Begriff Alkoxycarbonyl in den Definitionen von $R^2$ und $R^3$ in den allgemeinen Formeln ist geradkettiges oder verzweigtes Alkoxycarbonyl mit 1 bis 6, vorzugsweise 1 bis 4 Kohlenstoffatomen im Alkoxyrest zu verstehen. Beispielhaft seien genannt: Methoxycarbonyl, Ethoxycarbonyl, n-Propoxycarbonyl, i-Propoxycarbonyl, n-, i-, s- und t-Butoxycarbonyl.

Unsubstituiertes oder substituiertes Aryloxy und Arylthio stehen im allgemeinen in der Definition von $R^1$ in den allgemeinen Formeln für Aryl mit vorzugsweise 6 bis 10 Kohlenstoffatomen im Arylteil. Beispielhaft seien unsubstituiertes oder substituiertes Phenoxy oder Phenylthio genannt.

Unsubstituiertes oder substituiertes Aralkyloxy oder Aralkylthio enthalten im allgemeinen in der Definition von $R^1$ vorzugsweise 1 bis 6 Kohlenstoffatome im geradkettigen oder verzweigten Alkylteil und vorzugsweise Phenyl als Arylteil. Als Aralkyloxy- oder -thiogruppen seien beispielhaft Benzyloxy und Benzylthio genannt.

Halogen steht in den Definitionen von $R^1$, $R^2$, $R^3$ und $R^4$ in den allgemeinen Formeln für Fluor, Chlor, Brom, Iod, vorzugsweise für Fluor, Chlor und Brom.

Die Substituenten für die Arylreste als solche oder in Zusammensetzungen wie Aryloxy, Arylthio, Aralkyloxy und Aralkylthio haben die im folgenden angegebenen Bedeutungen.

Halogen steht im allgemeinen als Substituent für Fluor, Chlor, Brom und Iod, bevorzugt für Fluor, Chlor und Brom, besonders bevorzugt für Fluor und Chlor.

Alkyl steht im allgemeinen als Substituent oder in Zusammensetzungen wie Phenylalkylthio oder Phenylalkyloxy für geradkettiges oder verzweigtes Alkyl, bevorzugt mit 1 bis 6, besonders bevorzugt mit 1 bis 4 Kohlenstoffatomen, ganz besonders bevorzugt sind Methyl und Ethyl. Die beispielhafte Aufzählung entspricht der weiter oben gegebenen.

Alkoxy steht im allgemeinen als Substituent für geradkettiges oder verzweigtes Alkoxy mit 1 bis 6, besonders bevorzugt 1 bis 4 Kohlenstoffatomen je Alkylrest; beispielhaft und vorzugsweise seien genannt: Methoxy, Ethoxy, n- und i-Propoxy, n-, i-, s- und t-Butoxy, n-Hexoxy und i-Hexoxy.

Alkylthio steht im allgemeinen als Substituent in den Resten für geradkettiges oder verzweigtes Alkylthio mit vorzugsweise 1 bis 6 Kohlenstoffatomen, beispielsweise sind darunter die folgenden Gruppen zu verstehen: Methylthio, Ethylthio, Propylthio, Butylthio, Pentylthio sowie ihre Isomeren, wie z.B. i-Propylthio, i-, s- und t-Butylthio, 1-Methyl-butylthio, 2-Methyl-butylthio- und 3-Methyl-butylthio. Bevorzugte Alkylthioreste enthalten 1 bis 4 Kohlenstoffatome. Besonders bevorzugt sind Methylthio, Ethylthio, n-, i-, s-Propylthio und n-, i-, s- und t-Butylthio.

Alkoxycarbonyl steht im allgemeinen als Substituent in den Resten für geradkettiges oder verzweigtes Alkoxycarbonyl mit 1 bis 6, vorzugsweise 1 bis 4 Kohlenstoffatomen im Alkoxyrest; beispielhaft seien genannt: Methoxycarbonyl, Ethoxycarbonyl, n-Propoxycarbonyl, i-Propoxycarbonyl, n-, i-, s- und t-Butoxycarbonyl.

Cycloalkyl steht im allgemeinen als Substituent in den Resten für Cycloalkyl mit vorzugsweise 3 bis 7, insbesondere 3, 5 oder 6 Kohlenstoffatomen. Beispielhaft seien unsubstituiertes oder substituiertes Cyclopropyl, Cyclobutyl, Cyclopentyl, Cyclohexyl und Cycloheptyl genannt.

Alkenyl und Alkinyl stehen im allgemeinen als Substituenten in den Resten für geradkettiges oder verzweigtes Alkenyl oder Alkinyl mit vorzugsweise 2 bis 6, besonders bevorzugt 2 bis 4 und ganz besonders bevorzugt 2 oder 3 Kohlenstoffatomen. Beispielhaft seien Vinyl, Allyl, Propenyl-(2)-, 1-Butenyl, 2-Butenyl, 3-Butenyl, Ethinyl, 1-Propinyl, 2-Propinyl, 1-Butinyl, 2-Butinyl, 3-Butinyl genannt.

Alkenyloxy und Alkinyloxy stehen im allgemeinen als Substituenten in den Resten für geradkettiges oder verzweigtes Alkenyloxy oder Alkinyloxy mit vorzugsweise 2 bis 6, besonders bevorzugt 2 bis 4 und ganz besonders bevorzugt mit 3 Kohlenstoffatomen. Beispielhaft seien Alkyloxy, 2-Propenyloxy, 2-Butenyloxy, 2-Propinyloxy, 2-Butinyloxy genannt.

Halogenalkyl und Halogenalkoxy stehen im allgemeinen als Substituenten in den Resten für geradkettiges oder verzweigtes Halogenalkyl oder Halogenalkoxy mit je 1 bis 6 Kohlenstoffatomen, besonders bevorzugt mit 1 bis 4 Kohlenstoffatomen und jeweils 1 bis 13, vorzugsweise 1 bis 9, gleichen oder verschiedenen Halogenatomen wie unter Halogen definiert; beispielhaft seien genannt: Fluormethyl, Chlormethyl, Brommethyl, Fluorethyl, Chlorethyl, Bromethyl, Fluor-n-propyl, Chlor-n-propyl, Difluormethyl, Trifluormethyl, Dichlormethyl, Trichlormethyl, Difluorethyl, Trifluorethyl, Trichlorethyl, Chlor-difluor-methyl, Trifluorchlorethyl, Chlorbutyl, Fluorbutyl, Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy und Trifluorchlorethoxy.

Halogenalkylthio steht im allgemeinen als Substituent in den Resten für geradkettige oder verzweigtes Halogenalkylthio mit je 1 bis 6 Kohlenstoffatomen, besonders bevorzugt mit 1 bis 4 Kohlenstoffatomen und jeweils 1 bis 13, vorzugsweise 1 bis 9, gleichen oder verschiedenen Halogenatomen wie unter Halogen definiert; beispielhaft seien genannt: Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Brombutylthio, Fluor-i-propylthio, Chlor-i-propylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

(Di)alkylamino steht im allgemeinen als Substituent in den Resten für eine Aminogruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten,wobei Methyl, Ethyl, n- und i-Propyl genannt seien. Beispielhaft seien Methylamino, Ethylamino, n-Propylamino, i-Propylamino, Dimethylamino, Diethylamino, Di-n-propylamino und Di-i-propylamino aufgeführt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, N-Methylpiperazino, Thiomorpholino und 2,6-Dimethylmorpholino.

(Di)alkylaminocarbonyl steht im allgemeinen als Substituent in den Resten für eine Aminocarbonylgruppe mit 1 oder 2 Alkylgruppen, welche jeweils geradkettig oder verzweigt, gleich oder verschieden sein können und vorzugsweise jeweils 1 bis 6, insbesondere 1 bis 4 Kohlenstoffatome enthalten, wobei Methyl, Ethyl, n- und i-Propyl genannt seien. Beispielhaft seien Methylaminocarbonyl, Ethylaminocarbonyl, Dimethylaminocarbonyl und Diethylaminocarbonyl genannt.

Wenn im Falle von Dialkylamino die beiden Reste gemeinsam mit dem Stickstoffatom, an welches sie gebunden sind, einen Ring bilden, enthält dieser 5 bis 7, vorzugsweise 5 oder 6 Ringglieder. Der Ring kann 1 bis 3 Doppelbindungen enthalten, vorzugsweise ist er jedoch gesättigt. Der Ring kann ein weiteres Heteroatom, vorzugsweise Sauerstoff, Schwefel oder Stickstoff enthalten und durch 1 oder 2 Alkylgruppen vorzugsweise durch eine Alkylgruppe substituiert sein. Beispielhaft seien genannt: Pyrrolidino, Piperidino, Morpholino, n-Methylpiperazino, Thiomorpholino und 2,6-Dimethylmorpholino.

Alkylcarbonylamino steht im allgemeinen als Substituent in den Resten für geradkettiges oder verzweigtes Alkylcarbonylamino mit 1 bis 6, vorzugsweise 1 bis 4 Kohlenstoffatomen im Alkylrest; beispielhaft seien

genannt: Methylcarbonylamino, Ethylcarbonylamino, n-Propylcarbonylamino, i-Propylcarbonylamino.

Alkylhydrazo steht im allgemeinen als Substituent in den Resten für geradkettiges oder verzweigtes Alkylhydrazo mit 1 bis 6, vorzugsweise 1 bis 4 Kohlenstoffatomen im Alkylteil. Beispielhaft seien genannt: 2-Methylhydrazo, 2-Ethylhydrazo, 2-Propylhydrazo und 2,2-Dimethylhydrazo.

Die erfindungsgemäßen Pyridinylpyrimidin-Derivate sind durch die Formel (I) allgemein definiert. Bevorzugt sind Verbindungen der Formel (I), bei welchen

$R^1$ für Wasserstoff, Halogen, jeweils geradkettiges oder verzweigtes Alkoxy oder Alkylthio mit jeweils 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen , Amino, Alkyl- oder Dialkylamino mit jeweils 1 bis 6 Kohlenstoffatomen in den jeweiligen geradkettigen oder verzweigten Alkylteilen steht, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist; weiterhin für jeweils gegebenenfalls einfach oder mehrfach, gleich oder verschieden substituiertes Phenoxy oder Phenylthio, steht, wobei als Phenylsubstituenten genannt seien: Halogen, Cyano, Nitro, Alkyl, Alkoxy oder Alkylthio mit jeweils 1 oder 2 Kohlenstoffatomen, sowie Halogenalkyl, Halogenalkoxy oder Halogenalkylthio mit jeweils 1 oder 2 Kohlenstoffatomen und jeweils 1 bis 5 Fluor- und/oder Chloratomen oder $R^1$ für jeweils gegebenenfalls einfach oder mehrfach, gleich oder verschieden substituiertes Phenylalkoxy oder Phenylalkylthio mit jeweils 1 bis 4 Kohlenstoffatomen im jeweiligen, geradkettigen oder verzweigten Alkylteil steht, wobei als Substituenten des Phenylteils die oben genannten Phenylsubstituenten infrage kommen,

$R^2$ und $R^3$ unabhängig voneinander, gleich oder verschieden, jeweils für Wasserstoff, Halogen, geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxy oder Alkylthio mit jeweils 1 bis 6 Kohlenstoffatomen, oder für geradkettiges oder verzweigtes Alkoxycarbonyl mit 1 bis 6 Kohlenstoffatomen im Alkoxyteil stehen, oder

$R^2$ und $R^3$ gemeinsam für eine Alkylkette mit 3 bis 5 Kohlenstoffatomen stehen, die über die Ringpositionen 3 und 4 oder 4 und 5 verbunden ist,

$R^4$ für Wasserstoff, Halogen, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, Hydroxy, Mercapto, jeweils geradkettiges oder verzweigtes Alkoxy oder Alkylthio mit jeweils 1 bis 6 Kohlenstoffatomen, Amino, Alkyl- oder Dialkylamino mit jeweils 1 bis 6 Kohlenstoffatomen in den jeweiligen, geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, oder für jeweils geradkettiges oder verzweigtes Alkenyloxy oder Alkinyloxy mit jeweils 2 bis 6 Kohlenstoffatomen steht,

$R^5$ für Wasserstoff, geradkettiges oder verzweigtes Alkyl mit 1 bis 6 Kohlenstoffatomen steht und

Ar für gegebenenfalls einfach oder mehrfach, gleich oder verschieden substituiertes Phenyl steht, wobei als Substituenten genannt seien: Halogen, Cyano, Nitro, Hydroxy, Amino, Mercapto, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy, mit jeweils 1 bis 6 Kohlenstoffatomen und jeweils 1 bis 13 gleichen oder verschiedenen Halogenatomen, jeweils geradkettiges oder verzweigtes Alkenyl oder Alkinyl mit 2 bis 6 Kohlenstoffatomen, jeweils geradkettiges oder verzweigtes Alkenyloxy oder Alkinyloxy mit 2 bis 6 Kohlenstoffatomen, Cycloalkyl mit 3 bis 8 Kohlenstoffatomen, Phenyl, Phenoxy, Phenylthio, Phenylalkylthio oder Phenylalkyloxy mit jeweils 1 bis 4 Kohlenstoffatomen im geradkettigen oder verzweigten Alkylteil,

weiterhin seien als Phenylsubstituenten genannt:
(Di)alkylamino mit 1 bis 6 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, oder weiterhin Phenylamino, gegebenenfalls 1- oder 2-fach durch geradkettiges oder verzweigtes Alkyl mit 1 bis 6 Kohlenstoffatomen oder Phenyl substituiertes Hydrazino, Alkyl- oder Phenylcarbonylamino mit 1 bis 6 Kohlenstoffatomen im geradkettigen oder verzweigten Alkylteil, Aminocarbonyl, (Di)alkylaminocarbonyl mit 1 bis 6 Kohlenstoffatomen in den jeweiligen geradkettigen oder verzweigten Alkylteilen wobei im Falle von Dialkylaminocarbonyl die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel-oder Stickstoffatom enthält und welcher gegebenenfalls durch 1 oder 2 Alkylgruppen mit 1 oder 2 Kohlenstoffatomen substituiert ist sowie geradkettiges oder verzweigtes Alkoxycarbonyl mit 1 bis 6 Kohlenstoffatomen, oder zwei benachbarte Phenylsubstituenten stehen gemeinsam für Alkandiyl mit 1 bis 6 Kohlenstoffatomen, welches gegebenenfalls ein oder mehrere Heteroatome aus der Reihe Sauerstoff, Schwefel und Stickstoff enthält.

Besonders bevorzugt sind Verbindungen der Formel (I), bei welchen

$R^1$ für Wasserstoff, Fluor, Chlor, Brom, jeweils geradkettiges oder verzweigtes Alkoxy oder Alkylthio mit jeweils 1 bis 4 Kohlenstoffatomen, für geradkettiges oder verzweigtes Halogenalkyl mit 1 bis 4 Kohlenstoffatomen und 1 bis 9 Fluor- und/oder Chloratomen für Amino, Alkyl- oder Dialkylamino mit jeweils 1 bis 4 Kohlenstoffatomen in den jeweiligen geradkettigen oder verzweigten Alkylteilen steht, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist, weiterhin für jeweils gegebenenfalls ein- oder zweifach, gleich oder verschieden substituiertes Phenoxy oder Phenylthio, wobei als Phenylsubstituenten genannt seien: Fluor, Chlor, Brom, Cyano, Nitro, Methyl, Trifluormethyl, Methoxy, Trifluormethoxy, Methylthio oder Trifluormethylthio, oder $R^1$ für jeweils gegebenenfalls ein- oder zweifach, gleich oder verschieden substituiertes Phenylalkoxy oder Phenylalkylthio mit jeweils 1 oder 2 Kohlenstoffatomen im Alkylteil steht, wobei als Substituenten des Phenylteils die oben genannten Phenylsubstituenten infrage kommen,

$R^2$ für Wasserstoff, Fluor, Chlor, Brom, für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, für jeweils geradkettiges oder verzweigtes Alkoxy oder Alkylthio mit 1 bis 4 Kohlenstoffatomen, oder für geradkettiges oder verzweigtes Alkoxycarbonyl mit 1 bis 4 Kohlenstoffatomen im Alkoxyteil steht,

$R^3$ für Wasserstoff, Fluor, Chlor, Brom, geradkettiges oder verzweigtes Alkyl mit 1 bis 4 Kohlenstoffatomen steht oder

$R^2$ und $R^3$ gemeinsam für eine Alkylkette mit 3 oder 4 Kohlenstoffatomen stehen, die über die Ringpositionen 3 und 4 oder 4 und 5 verbunden ist,

$R^4$ für Wasserstoff, Fluor, Chlor, Brom, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, Hydroxy, Mercapto, jeweils geradkettiges oder verzweigtes Alkoxy oder Alkylthio mit 1 bis 4 Kohlenstoffatomen, Amino, Alkyl- oder Dialkylamino mit jeweils 1 bis 4 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist, oder für jeweils geradkettiges oder verzweigtes Alkenyloxy oder Alkinyloxy mit jeweils 3 bis 4 Kohlenstoffatomen steht,

$R^5$ für Wasserstoff, geradkettiges oder verzweigtes Alkyl mit 1 bis 4 Kohlenstoffatomen steht und

Ar für gegebenenfalls 1- bis 3-fach, gleich oder verschieden substituiertes Phenyl steht, wobei als Substituenten genannt seien: Fluor, Chlor, Brom, Iod, Cyano, Nitro, Hydroxy, Mercapto, Amino, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, jeweils geradkettiges oder verzweigtes Alkoxy oder Alkylthio mil jeweils 1 bis 4 Kohlenstoffatomen, jeweils geradkettiges oder verzweigtes Halogenalkoxy oder Halogenalkylthio mit jeweils 1 bis 4 Kohlenstoffatomen und jeweils 1 bis 9 Fluor-und/oder Chloratomen, jeweils geradkettiges oder verzweigtes Alkenyl oder Alkinyl mit jeweils 2 bis 4 Kohlenstoffatomen, jeweils geradkettiges oder verzweigtes Alkenyloxy oder Alkinyloxy mit jeweils 3 bis 4 Kohlenstoffatomen, Cycloalkyl mit 3 bis 6 Kohlenstoffatomen, Phenyl, Phenoxy, Phenylthio, Phenylalkoxy oder Phenylalkylthio mit jeweils 1 oder 2 Kohlenstoffatomen im Alkylteil; weiterhin seien als Phenylsubstituenten genannt:
(Di)alkylamino mit 1 bis 4 Kohlenstoffatomen in den jeweiligen geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist; weiterhin Phenylamino, gegebenenfalls 1- oder 2-fach durch geradkettiges oder verzweigtes Alkyl mit 1 bis 4 Kohlenstoffatomen oder Phenyl substituiertes Hydrazino, Alkyl- oder Phenylcarbonylamino mit 1 bis 4 Kohlenstoffatomen im geradkettigen oder verzweigten Alkylteil, Aminocarbonyl, (Di-)alkylaminocarbonyl mit 1 bis 4 Kohlenstoffatomen in den jeweiligen geradkettigen oder verzweigten Alkylteilen, wobei im Falle von Dialkylaminocarbonyl die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist; sowie geradkettiges oder verzweigtes Alkoxycarbonyl mit 1 bis 4 Kohlenstoffatomen oder zwei benachbarte Phenylsubstituenten stehen gemeinsam für Alkandiyl mit 1 bis 4 Kohlenstoffatomen, welches gegebenenfalls 1 oder 2 Heteroatome aus der Reihe Sauerstoff, Schwefel und Stickstoff enthält.

Ganz besonders bevorzugt sind Verbindungen der Formel (I), bei welchen

$R^1$ für Wasserstoff, Fluor, Chlor, Methoxy, Ethoxy, n-Propoxy oder i-Propoxy, Methylthio, Ethylthio, Trifluormethyl, Trichlormethyl, Difluormethyl oder Chlorfluormethyl, für Methylamino, Dimethylamino, Ethylamino, Diethylamino, Pyrrolidino, Piperidino, Morpholino, Thiomorpholino, n-Methylpiperazino, 2,6-Dimethylmorpholino, für Phenoxy, Phenylthio, Benzyloxy oder Benzylthio steht,

$R^2$ für Wasserstoff, Fluor, Chlor, Brom, Methyl, Ethyl, n-Propyl oder i-Propyl, für Trifluormethyl, Trichlormethyl, Difluormethyl, Dichlormethyl, für Methoxy oder Ethoxy, für Methylthio, für Methoxy- oder Ethoxycarbonyl steht,

$R^3$ für Wasserstoff, Fluor, Chlor, Brom, Methyl, Ethyl, n-Propyl, i-Propyl oder t-Butyl steht,

$R^4$ für Wasserstoff, Fluor, Chlor, Brom, Methyl, Trifluormethyl, Trichlormethyl, Hydroxy, Mercapto, Methoxy, Ethoxy, n-Propoxy, i-Propoxy, Methylthio, Ethylthio, n-Propylthio oder i-Propylthio, n-Butylthio, i-Butylthio, s-Butylthio oder t-Butylthio, für Amino, Methylamino, Ethylamino, Dimethylamino, Diethylamino, Pyrrolidino, Piperidino, Morpholino, Thiomorpholino, N-Methylpiperazino oder 2,6-Dimethylmorpholino, für 2-Propenyloxy oder 2-Propinyloxy steht,

$R^5$ für Wasserstoff, Methyl, Ethyl, n-Propyl, i-Propyl oder n-Butyl steht und

Ar für gegebenenfalls 1- bis 3-fach, gleich oder verschieden substituiertes Phenyl steht, wobei als Substituenten genannt seien:
Fluor, Chlor, Brom, Iod, Cyano, Nitro, Hydroxy, Amino, Mercapto, Methyl, Ethyl, n-Propyl oder i-Propyl, n-Butyl, s-Butyl, i-Butyl oder t-Butyl, Trifluormethyl, Trichlormethyl, Difluormethyl, Methoxy, Ethoxy, Methylthio, Ethylthio, Trifluormethoxy, Difluormethoxy, Trifluormethylthio, Chlordifluormethylthio, Vinyl, 2-Propenyl, 2-Propinyl, 2-Propenyloxy, 2-Propinyloxy, Cyclopropyl, Cyclopentyl, Cyclohexyl, Phenyl, Phenoxy, Phenylthio, Phenylmethoxy oder Phenylmethylthio, weiterhin seien als Phenylsubstituenten genannt:
Methylamino, Dimethylamino, Ethylamino, Diethylamino, Pyrrolidino, Piperidino, Morpholino, Thiomorpholino, N-Methylpiperazino, 2,6-Dimethylmorpholino, Phenylamino, Hydrazino, 2-Methylhydrazo, 2,2-Dimethylhydrazo, 2-Phenylhydrazo, Methylcarbonylamino, Phenylcarbonylamino, Aminocarbonyl, Methylaminocarbonyl, Dimethylaminocarbonyl, Methoxycarbonyl oder Ethoxycarbonyl.

Im einzelnen seien außer den bei den Herstellungsbeispielen genannten Verbindungen die folgenden Pyridinylpyrimidin-Derivate der allgemeinen Formel (I) genannt:

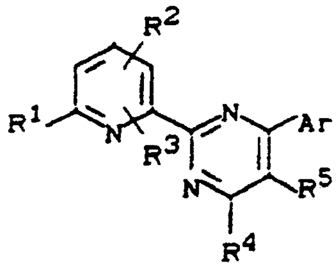

(I)

**Tabelle 1:**

| $R^1$ | $R^2$ | $R^3$ | $R^4$ | $R^5$ | Ar |
|---|---|---|---|---|---|
| Cl | H | H | $CH_3O-$ | $CH_3$ | 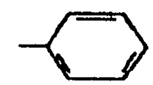 |
| Cl | H | H | $C_2H_5O-$ | H | 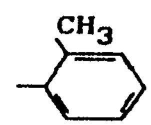 |
| Cl | H | H | $-OCH_2-CH=CH_2$ | H | 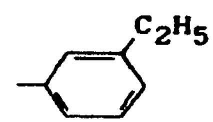 |
| Cl | 5-Cl | H | $-OCH_2-C\equiv CH$ | H | 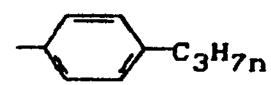 |
| Cl | 4-Cl | H | $CH_3$ | H | 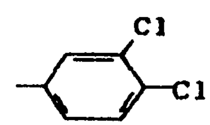 |
| Cl | 5-Cl | 3-Cl | H | $CH_3$ | 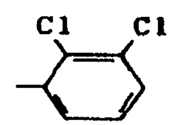 |

Tabelle 1 - Fortsetzung

| $R^1$ | $R^2$ | $R^3$ | $R^4$ | $R^5$ | Ar |
|---|---|---|---|---|---|
| Br | H | H | Br | $-C_2H_5$ | 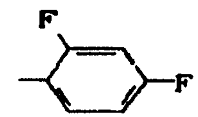 |
| Br | H | H | OH | $CH_3$ | 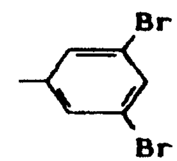 |
| Br | $5-CF_3$ | H | H | H | 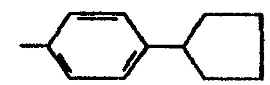 |
| Br | H | $3-CO_2CH_3$ | $CF_3$ | H | 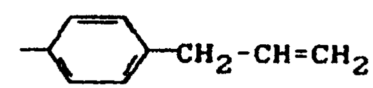 |
| F | 4-F | H | $-SCH_3$ | H | 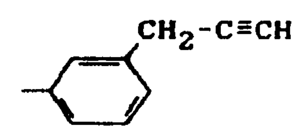 |
| F | $5-SCH_3$ | H | $-NHCH_3$ | H | 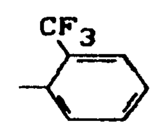 |

<u>Tabelle 1</u> - Fortsetzung

| $R^1$ | $R^2$ | $R^3$ | $R^4$ | $R^5$ | Ar |
|---|---|---|---|---|---|
| $CH_3O-$ | H | H | $-N(CH_3)_2$ | H | 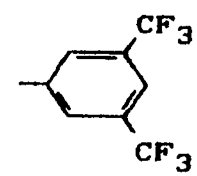 |
| $CH_3O-$ | H | $3-CH_3$ | $-NH_2$ | H | 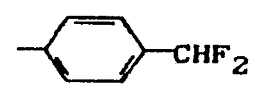 |
| $CH_3O-$ | $5-C_2H_5$ | H | -SH | H | 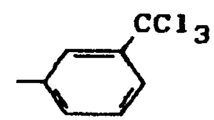 |
| $CH_3O-$ | H | H | Cl | H | 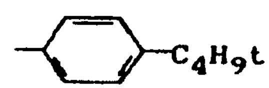 |
| $C_2H_5O-$ | $4-C_2H_5O$ | H | Cl | H | 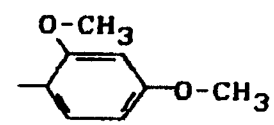 |
| $C_2H_5O-$ | 5-Cl | H | F | H | 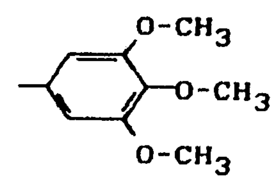 |

<u>Tabelle 1</u> - Fortsetzung

| $R^1$ | $R^2$ | $R^3$ | $R^4$ | $R^5$ | Ar |
|---|---|---|---|---|---|
| n-$C_3H_7O$- | H | H | $CH_3O$- | H | 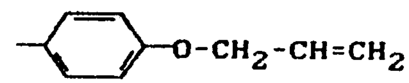 |
| $-SCH_3$ | H | H | $C_2H_5O$- | H | 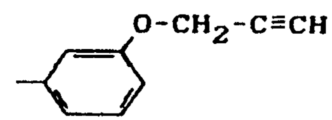 |
| $-SC_2H_5$ | H | H | $-CF_3$ | H | 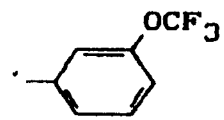 |
| $-N(CH_3)_2$ | H | H | $-CCl_3$ | H | 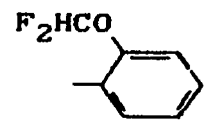 |
| $-CF_3$ | H | H | n-$C_3H_7O$- | H | 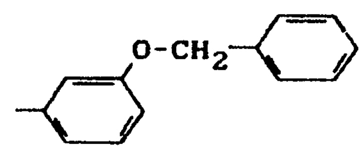 |

Tabelle 1 - Fortsetzung

| $R^1$ | $R^2$ | $R^3$ | $R^4$ | $R^5$ | Ar |
|---|---|---|---|---|---|
| $-CF_3$ | H | $3-OCH_3$ | OH | H | 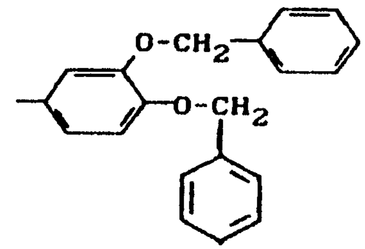 |
| $-CHF_2$ | H | H | $CH_3$ | $CH_3$ | 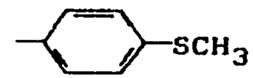 |
| $-CCl_3$ | H | H | H | $C_2H_5$ | 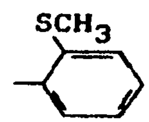 |
| -CHClF | H | 3-Cl | $CH_3S-$ | H | 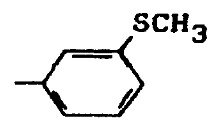 |
| H | $5-CH_3O-$ | $3-CH_3O-$ | SH | H | 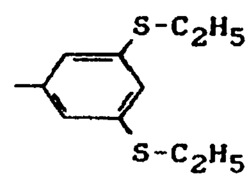 |

Tabelle 1 - Fortsetzung

| $R^1$ | $R^2$ | $R^3$ | $R^4$ | $R^5$ | Ar |
|---|---|---|---|---|---|
| 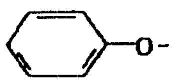 | H | H | $-NH_2$ | H | 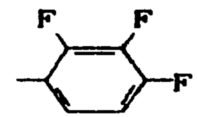 |
| 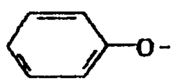 | H | H | $CH_3O-$ | H | 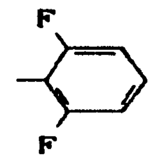 |
| 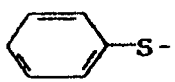 | H | H | $CH_3O-$ | $CH_3$ | 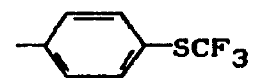 |
| 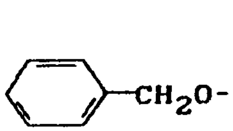 | H | H | $C_2H_5O-$ | H | 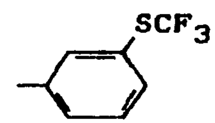 |
| 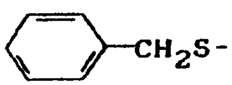 | H | H | Cl | H | 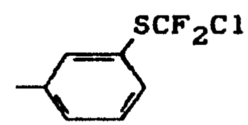 |
| 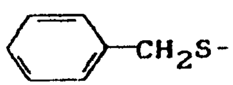 | H | $3-CF_3$ | Cl | H | 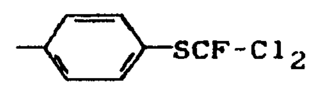 |

<u>Tabelle 1</u> - Fortsetzung

| $R^1$ | $R^2$ | $R^3$ | $R^4$ | $R^5$ | Ar |
|---|---|---|---|---|---|
| H | 5-Cl | 4-Cl | H | H | 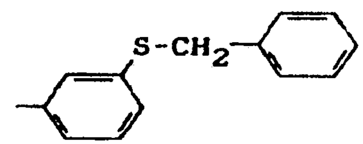 |
| H | 5-Cl | 3-Cl | H | H | 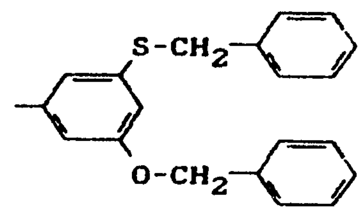 |
| H | H | 3-Br | H | H | 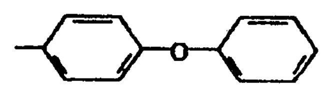 |
| H | H | 3-$CH_3$ | $CH_3$ | H | 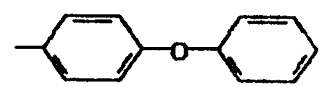 |
| H | H | 3-$CO_2$-$C_2H_5$ | $CH_3$ | H | 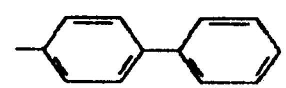 |

<u>Tabelle 1</u> - Fortsetzung

| $R^1$ | $R^2$ | $R^3$ | $R^4$ | $R^5$ | Ar |
|---|---|---|---|---|---|
| H | 5-$CO_2$-$CH_3$ | H | H | H | 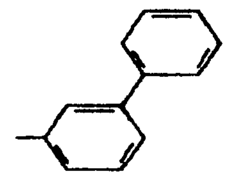 |
| H | 5-$CF_3$ | H | OH | H | 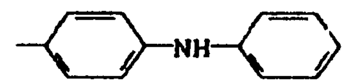 |
| Cl | 5-$CF_3$ | H | OH | H | 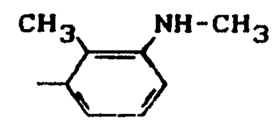 |
| $CH_3O$- | 5-$CF_3$ | H | Cl | H | 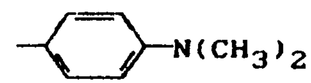 |
| Br | 5-$CHF_2$ | H | Cl | H | 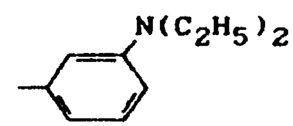 |
| Cl | 5-$CCl_3$ | H | Cl | H | 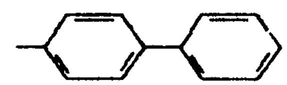 |

Tabelle 1 - Fortsetzung

| $R^1$ | $R^2$ | $R^3$ | $R^4$ | $R^5$ | Ar |
|---|---|---|---|---|---|
| $CH_3O-$ | 5-$CCl_3$ | H | Br | H | 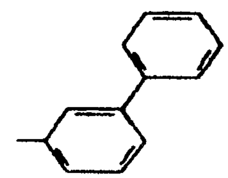 |
| F | 5-CHClF | H | H | $CH_3$ | 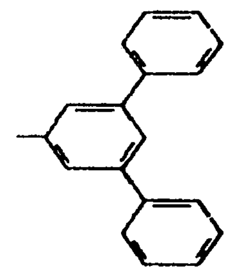 |
| $CH_3S-$ | 5-$CH_3$ | H | H | $CH_3$ | 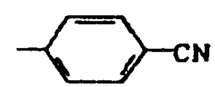 |
| H | 5-$C_2H_5$ | H | H | H | 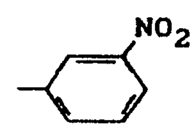 |
| H | 5-Cl | H | H | H | 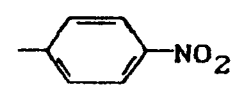 |

Tabelle 1 - Fortsetzung

| $R^1$ | $R^2$ | $R^3$ | $R^4$ | $R^5$ | Ar |
|---|---|---|---|---|---|
| Cl | 5-Cl | H | H | $C_2H_5$ | 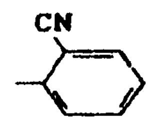 |
| $-N(CH_3)_2$ | 5-Cl | H | H | H | 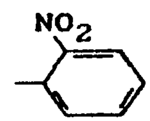 |
| 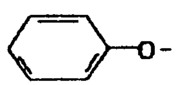 | 5-Cl | H | H | H | 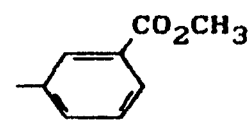 |
| 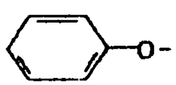 | 5-Br | H | H | H | 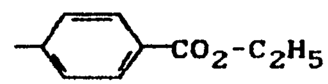 |
| 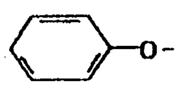 | 5-Br | H | $CH_3$ | H | 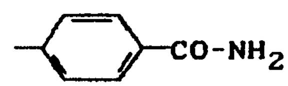 |
| 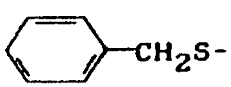 | 5-Br | H | $CH_3O$ | H | 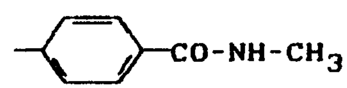 |

Tabelle 1 - Fortsetzung

| $R^1$ | $R^2$ | $R^3$ | $R^4$ | $R^5$ | Ar |
|---|---|---|---|---|---|
| H | 5-$CH_3O$- | H | $CH_3O$- | -$C_3H_7$-n | 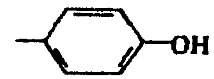 |
| -$CF_3$ | 5-$C_2H_5O$- | H | OH | H | 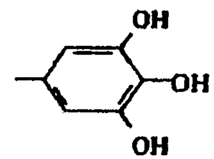 |
| H | H | 4-Br | H | H | 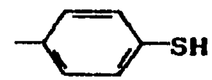 |
| H | H | 4-Br | $CH_3$ | H | 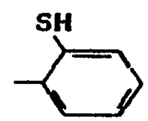 |
| H | H | 4-Cl | $CH_3O$- | H | 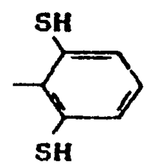 |
| H | H | 4-Cl | $CH_3O$- | H | 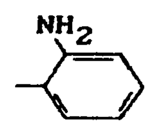 |

Tabelle 1 - Fortsetzung

| $R^1$ | $R^2$ | $R^3$ | $R^4$ | $R^5$ | Ar |
|---|---|---|---|---|---|
| H | H | $4-CF_3$ | $CH_3O-$ | H | 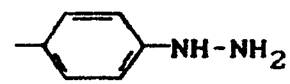 |
| H | H | $4-CHF_2$ | $n-C_3H_7S-$ | H | 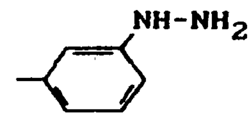 |
| H | H | $4-CHCl_2$ | $CH_2=CH-CH_2O-$ | H | 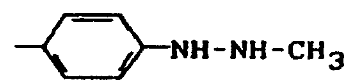 |
| H | 5-F | $4-CH_3$ | $n-C_3H_7O-$ | H | 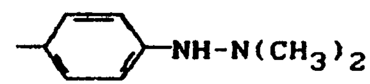 |
| H | $5-CH_3$ | $4-CH_3$ | $i-C_3H_7O-$ | H | 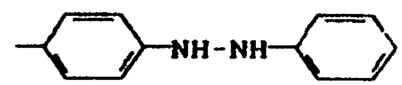 |
| H | H | $3-C_2H_5$ | $CH\equiv C-CH_2O-$ | H | 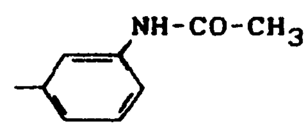 |

<u>Tabelle 1</u> - Fortsetzung

| $R^1$ | $R^2$ | $R^3$ | $R^4$ | $R^5$ | Ar |
|---|---|---|---|---|---|
| H | H | 3-$OCH_3$ | $CH_3S$- | H | 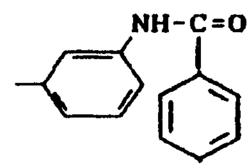 |
| H | H | 3-$OCH_3$ | $CH_3CN$- | H | 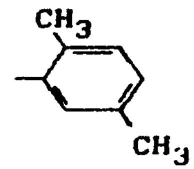 |
| H | H | 3-$OC_2H_5$ | $(CH_3)_2N$- | H | 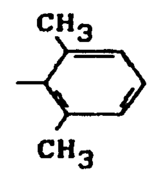 |
| H | H | 3-$C_3H_7$n | $CH_3O$- | H | 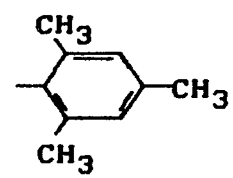 |
| H | H | 3-$CF_3$ | $CH_3O$- | $CH_3$ | 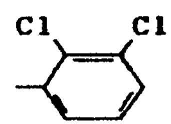 |

Tabelle 1 - Fortsetzung

| $R^1$ | $R^2$ | $R^3$ | $R^4$ | $R^5$ | Ar |
|---|---|---|---|---|---|
| H | H | 3-$CF_3$ | $CH_3O$- | -$C_2H_5$ | 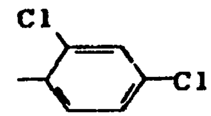 |
| H | H | 3-$CCl_3$ | $C_2H_5O$- | H | 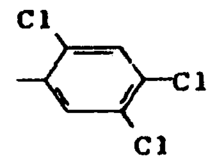 |
| H | H | 3-$CO_2$-$CH_3$ | SH | H | 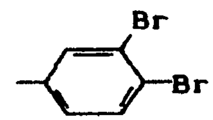 |
| H | H | 3-$CO_2$-$C_2H_5$ | $C_2H_5O$- | H | 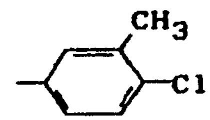 |
| H | H | 3-F | $CH_2$=CH-$CH_2O$- | H | 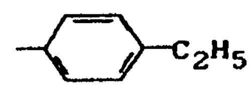 |
| H | 5-Cl | 3-Cl | H | $CH_3$ | 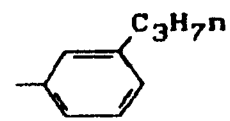 |

Tabelle 1 - Fortsetzung

| $R^1$ | $R^2$ | $R^3$ | $R^4$ | $R^5$ | Ar |
|---|---|---|---|---|---|
| H | 5-Br | 3-Br | H | H | 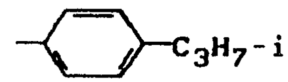 |
| H | 5-$CCl_3$ | 3-Cl | H | H | 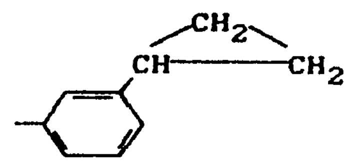 |
| H | 5-$CO_2$-$CH_3$ | 3-$C_2H_5O$- | OH | H | 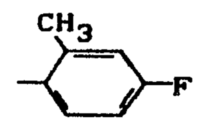 |
| H | H | H | OH | H | 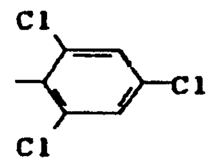 |
| H | H | H | Cl | H | 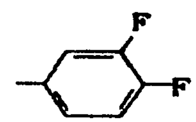 |
| H | H | H | Cl | H | 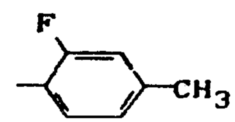 |

Tabelle 1 - Fortsetzung

| $R^1$ | $R^2$ | $R^3$ | $R^4$ | $R^5$ | Ar |
|---|---|---|---|---|---|
| H | H | H | Br | H | 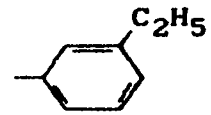 |
| Cl | H | H | F | H | 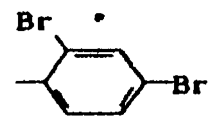 |
| Cl | H | H | H | $-C_3H_7$-n | 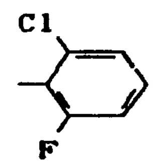 |
| Cl | H | H | H | H | 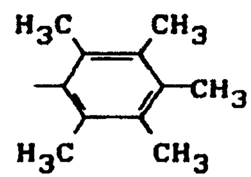 |
| Cl | H | H | H | H | 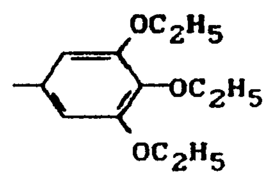 |

**Tabelle 1** - Fortsetzung

| $R^1$ | $R^2$ | $R^3$ | $R^4$ | $R^5$ | Ar |
|---|---|---|---|---|---|
| Br | H | H | H | $CH_3$ | 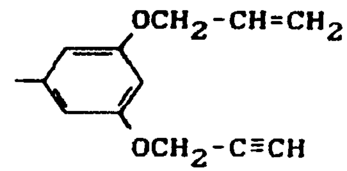 |
| Br | H | H | $CH_3O$ | H | 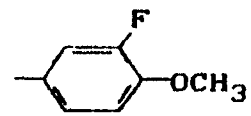 |
| $CH_3O-$ | H | H | H | H | 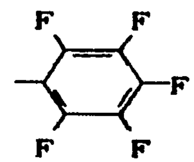 |
| $CH_3O-$ | H | H | H | H | 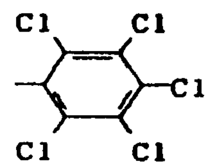 |
| $C_2H_5O-$ | H | H | H | H | 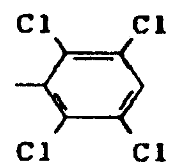 |

Tabelle 1 - Fortsetzung

| $R^1$ | $R^2$ | $R^3$ | $R^4$ | $R^5$ | Ar |
|---|---|---|---|---|---|
| n-$C_3H_7O$- | H | H | OH | OH | 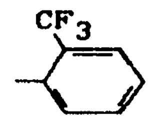 |
| i-$C_3H_7O$- | H | H | SH | H | 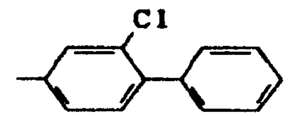 |
| -$CF_3$ | H | H | $NH_2$ | H | 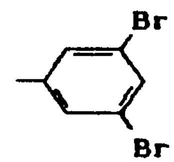 |
| $CH_3S$- | H | H | H | -$C_2H_5$ | 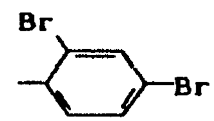 |
| $CH_3S$- | H | H | $CH_3$ | H | 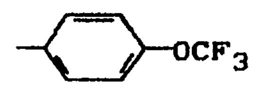 |
| $C_2H_5S$- | H | H | H | H | 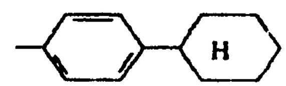 |

<u>Tabelle 1</u> - Fortsetzung

| $R^1$ | $R^2$ | $R^3$ | $R^4$ | $R^5$ | Ar |
|---|---|---|---|---|---|
| $C_2H_5S-$ | H | H | H | H | 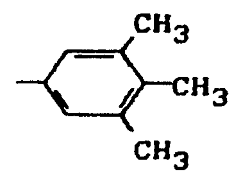 |
| $(CH_3)_2N-$ | H | H | H | H | 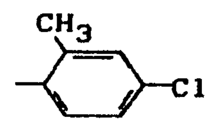 |
| $(C_2H_5)_2N-$ | H | H | H | H | 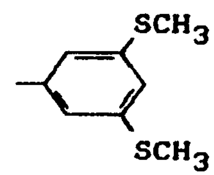 |
| $-CCl_3$ | H | H | Cl | H | 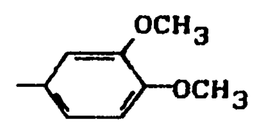 |
| $-CHF_2$ | H | H | H | H | 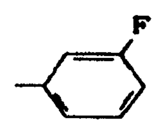 |

Tabelle 1 - Fortsetzung

| $R^1$ | $R^2$ | $R^3$ | $R^4$ | $R^5$ | Ar |
|---|---|---|---|---|---|
| 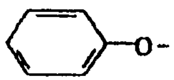 | H | H | H | H | 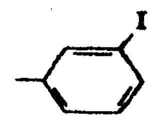 |
| 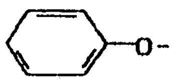 | H | H | H | $CH_3$ | 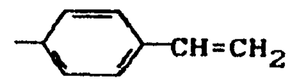 |
| 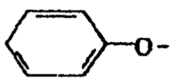 | H | H | OH | H | 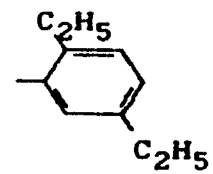 |
| 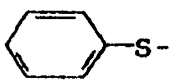 | H | H | OH | H | 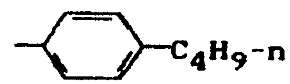 |
| 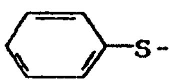 | H | H | Cl | H | 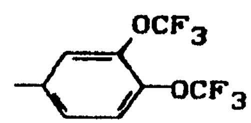 |
| 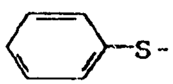 | H | H | $CH_3O$- | H | 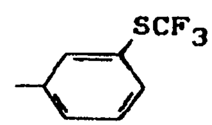 |

Tabelle 1 - Fortsetzung

| $R^1$ | $R^2$ | $R^3$ | $R^4$ | $R^5$ | Ar |
|---|---|---|---|---|---|
| 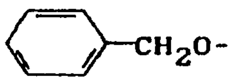 | H | H | OH | $C_2H_5$ | 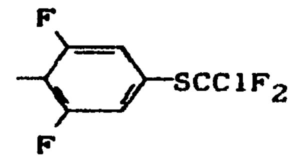 |
| 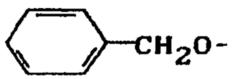 | H | H | $CH_3O-$ | H | 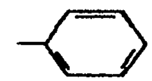 |
| 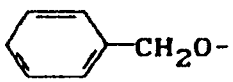 | H | H | $CH_3S-$ | H | 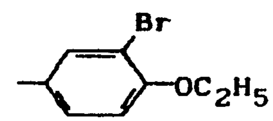 |
| 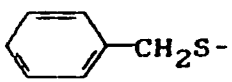 | H | H | Cl | H | 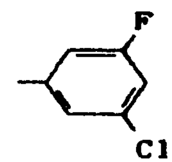 |
| 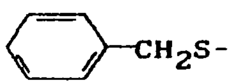 | H | H | F | H | 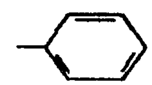 |
| 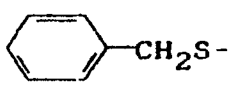 | H | H | H | H | 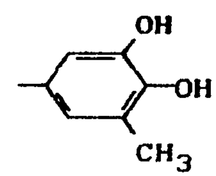 |

**Tabelle 1** - Fortsetzung

| $R^1$ | $R^2$ | $R^3$ | $R^4$ | $R^5$ | Ar |
|---|---|---|---|---|---|
| 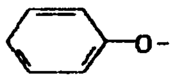 | H | H | H | H | 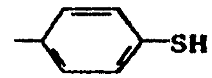 |
| F | H | H | H | $n-C_3H_7$ | 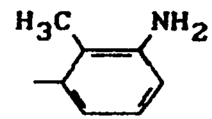 |
| F | H | H | $CH_3$ | H | 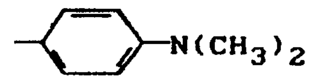 |
| Cl | H | H | $C_2H_5O-$ | H | 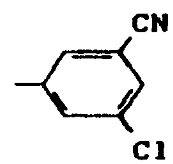 |
| Br | H | H | $CH_2=CH-CH_2O-$ | H | 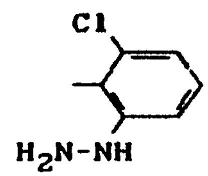 |
| H | H | H | $i-C_3H_7O-$ | H | 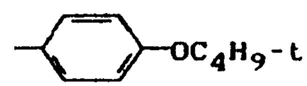 |

Tabelle 1 - Fortsetzung

| $R^1$ | $R^2$ | $R^3$ | $R^4$ | $R^5$ | Ar |
|---|---|---|---|---|---|
| 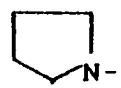 | H | H | H | H | 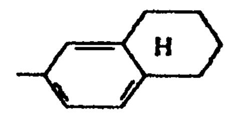 |
| 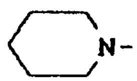 | H | H | H | $CH_3$ | 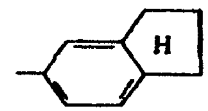 |
| H | 4,5-$(CH_2)_4$- | | H | H | 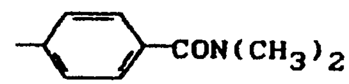 |
| H | H | 3-$CHCl_2$ | $CH_3$ | H | 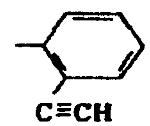 |
| 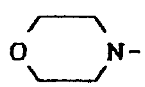 | H | H | H | $C_2H_5$ | 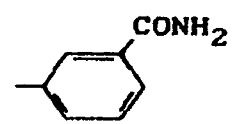 |
| H | 3-$CHF_2$ | H | $CH_3S$- | H | 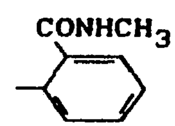 |

**Tabelle 1** - Fortsetzung

| $R^1$ | $R^2$ | $R^3$ | $R^4$ | $R^5$ | Ar |
|---|---|---|---|---|---|
| 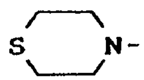 | 3,4-$(CH_2)_4$- | | $CH_3O$- | H | 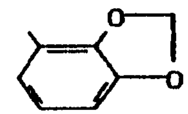 |
| 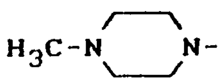 | H | H | $C_2H_5S$- | H | 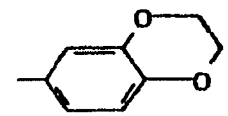 |
| H | 4-$C_2H_5$ | H | i-$C_3H_7S$- | H | 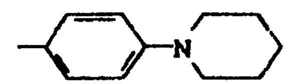 |
| H | 4,5-$(CH_2)_3$ | | H | $CH_3$ | 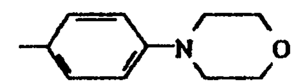 |
| H | 5-$CH_3$ | 3-$CH_3$ | H | H | 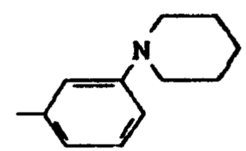 |
| 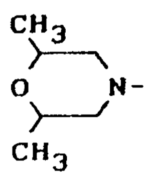 | H | H | $CH_3$ | H | 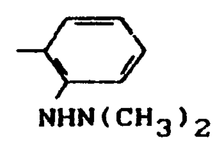 |

<u>Tabelle 1</u> - Fortsetzung

| $R^1$ | $R^2$ | $R^3$ | $R^4$ | $R^5$ | Ar |
|---|---|---|---|---|---|
| 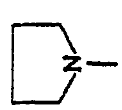 | H | 3-$CH_3$ | t-$C_4H_9S$- | H | 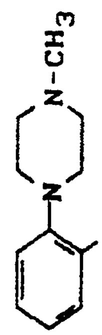 |
| $(C_2H_5)_2N$- | H | H | F | H | 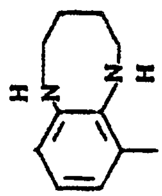 |
| H | 5-F | 3-F | $CH_3$ | $CH_3$ | 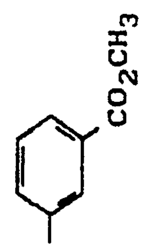 |
| 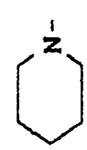 | H | n-$C_3H_7$ | Br | H | 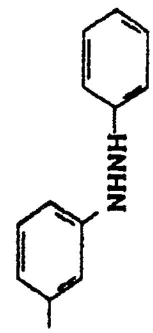 |

Verwendet man beispielsweise 4-Chlor-2-(2-pyridyl)-6-(4-fluor-phenyl)-pyrimidin als Ausgangsstoff und Wasserstoff sowie Palladium-Kohle als Katalysator, so läßt sich das erfindungsgemäße Verfahren (A) durch das folgende Formelschema beschreiben:

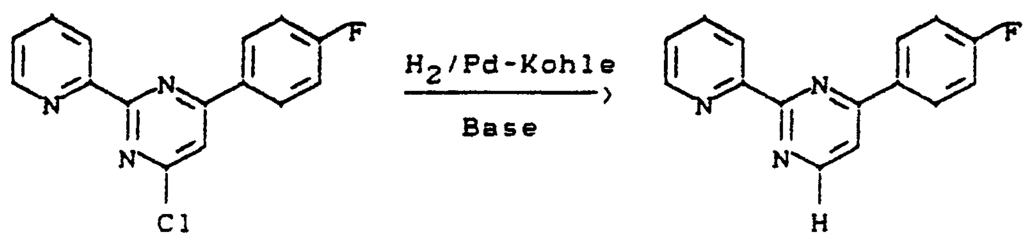


Verwendet man beispielsweise 4-Chlor-2-(2-pyridyl)-6-(4-fluor-phenyl)-pyrimidin und Natriummethanolat als Ausgangsstoffe, so kann das erfindungsgemäße Verfahren (B) durch das folgende Formelschema beschrieben werden:

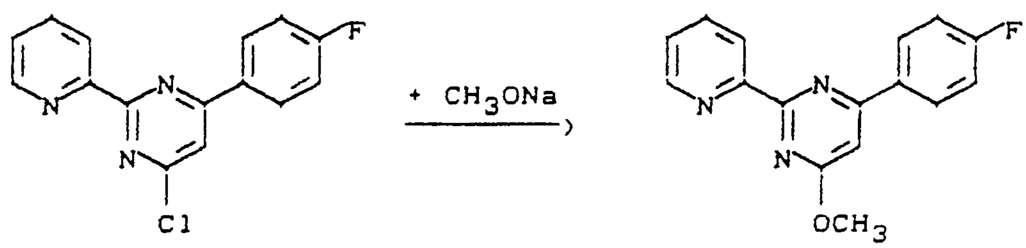


Verwendet man beispielsweise 4-Chlor-2-(2-pyridyl)-6-(4-fluor-phenyl)-pyrimidin und Malonsäurediethylester, so läßt sich das erfindungsgemäße Verfahren (C) durch das folgende Formelschema darstellen:

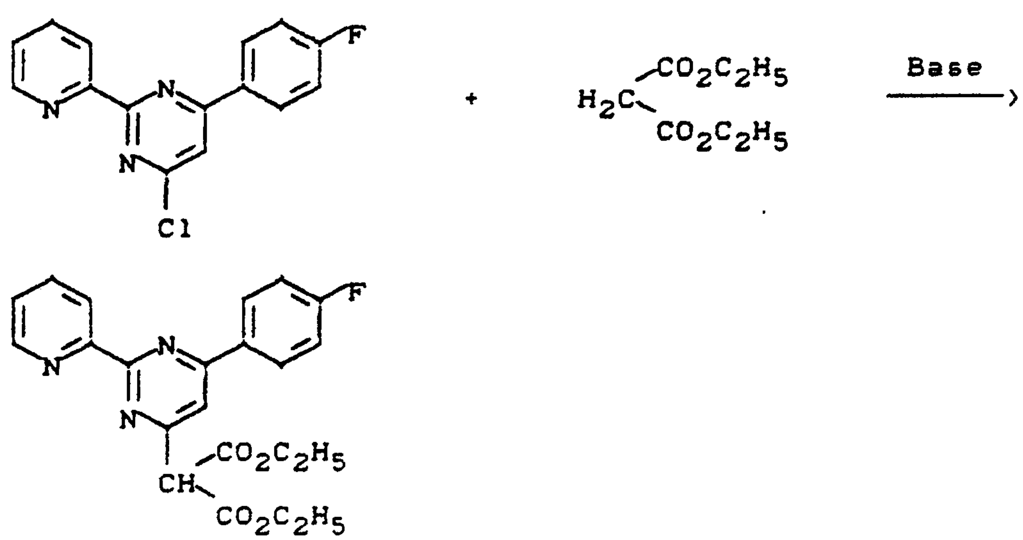


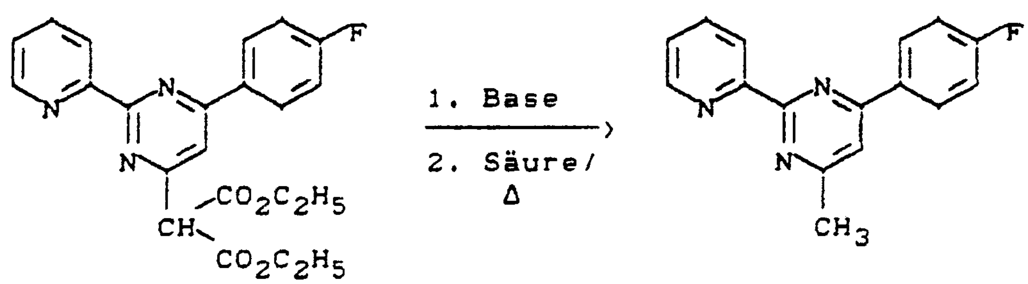

Verwendet man beispielsweise 2-Pyridinyl-amidin-Hydrochlorid und 1-(4-Cyclohexyl-phenyl)-3-dimethylamino-propen-on und Natriummethylat als Ausgangsstoffe, so läßt sich das erfindungsgemäße Verfahren (D-α) durch das folgende Formelschema darstellen:

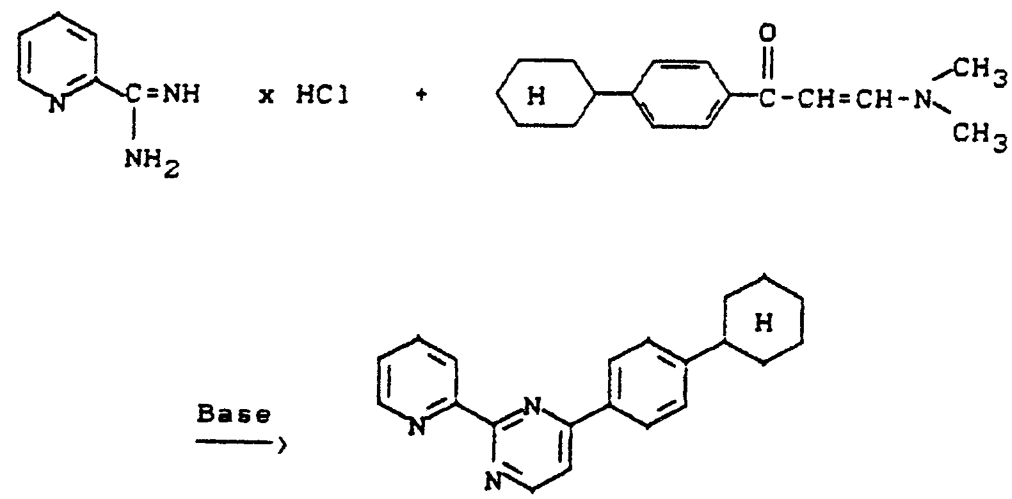


Verwendet man beispielsweise 2-Pyridinyl-amidin-Hydrochlorid und 1-Phenyl-3,3-dimethoxy-propan-1-on, so läßt sich das erfindungsgemäße Verfahren (D-ß) durch das folgende Formelschema beschreiben:

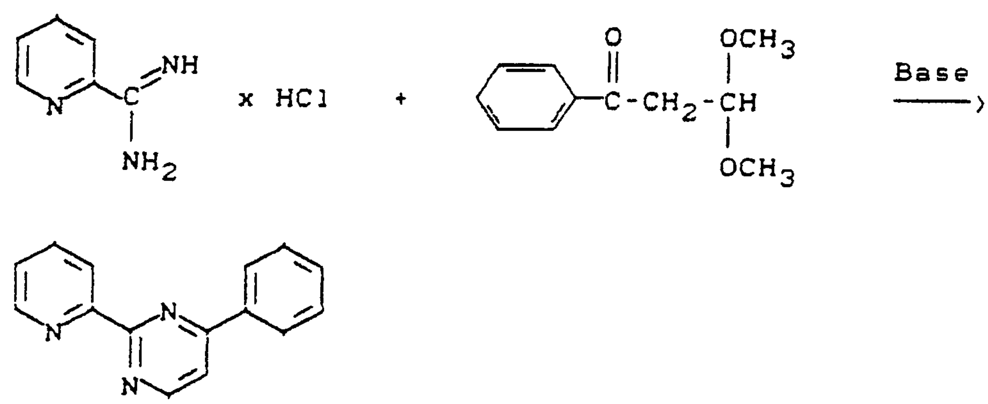


Verwendet man beispielsweise 2-Pyridinyl-amidin-Hydrochlorid und 1-Phenyl-1,3-butandion als Ausgangsstoffe, so läßt sich das erfindungsgemäße Verfahren (D-γ) durch das folgende Formelschema beschreiben:

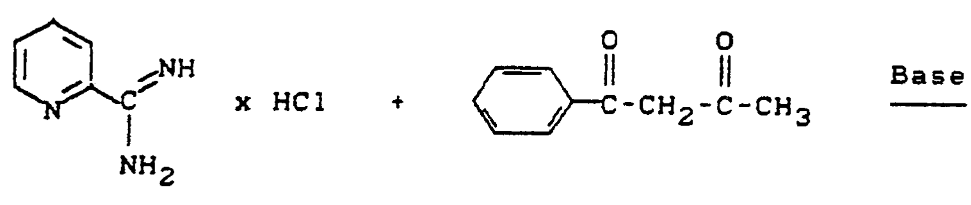

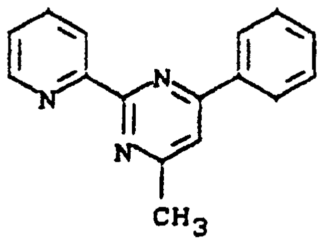

Verwendet man beispielsweise 2-Pyridinyl-amidin-Hydrochlorid und 1,1,3,3-Tetramethoxypropyl-benzol, so läßt sich das erfindungsgemäße Verfahren (D-δ) durch das folgende Formelschema beschreiben:

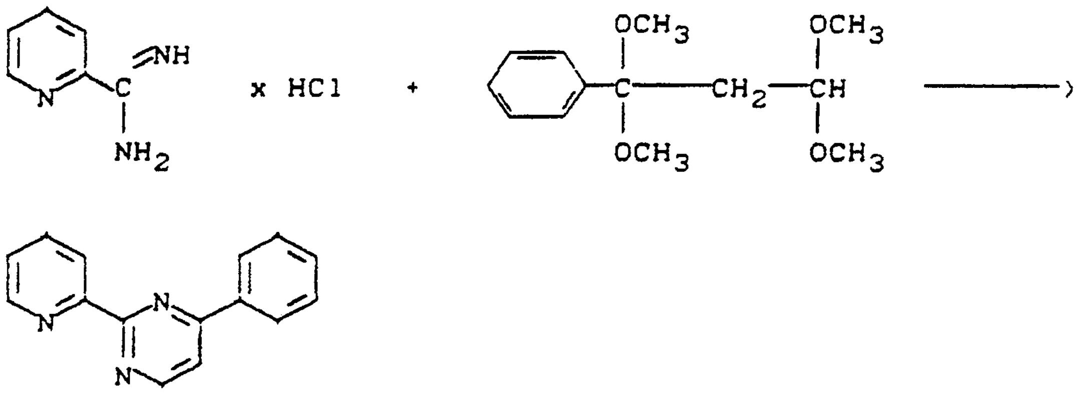

Die zur Durchführung der erfindungsgemäßen Verfahren (A), (B) und (C) als Ausgangsstoffe benötigten halogensubstituierten Pyridinylpyrimidin-Derivate sind durch die Formel (II) allgemein definiert. In dieser Formel (II) stehen $R^1$, $R^2$, $R^3$` $R^5$, Ar und X für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (II) sind neu. Sie lassen sich jedoch nach bekannten Verfahren in analoger Weise erhalten, indem man beispielsweise Picolinamin-Derivate der Formel (V)

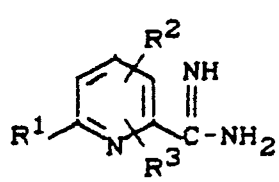

(V)

in welcher

$R^1$, $R^2$ und $R^3$ die oben angegbene Bedeutung haben, oder deren Säureadditionssalze mit Benzoylessigsäureester-Derivaten der Formel (X)

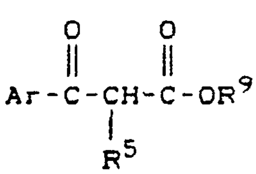

(X)

in welcher
Ar und $R^5$ die oben angegebene Bedeutung haben und
$R^9$ für Methyl oder Ethyl steht,
gegebenenfalls in Gegenwart eines Verdünnungsmittels wie beispielsweise Methanol, Ethanol, Dioxan, Tetrahydrofuran,Pyridin, N,N-Dimethylformamid, Wasser oder einer Mischung aus Wasser und den genannten Verdünnungsmitteln und gegebenenfalls in Gegenwart einer Base, wie beispielsweise Natriumhydroxid, Kaliumhydroxid, Kaliumcarbonat, Natriummethanolat, Kaliummethanolat, Triethylamin oder N,N-Diethylaninlin bei Temperaturen von 50 bis 150° C zu den ebenfalls neuen Verbindungen der Formel (XI)

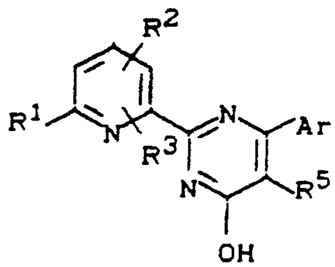

(XI)

in welcher
$R^1$, $R^2$, $R^3$, $R^5$ und Ar die oben angegebene Bedeutung haben,
cyclisiert und die so erhaltenen Verbindungen der Formel (XI) gegebenenfalls nach ihrer Isolierung, gegebenenfalls in Gegenwart eines Verdünnungsmittels, wie beispielsweise Benzol, Toluol oder Chlorbenzol mit einem Halogenierungsreagenz, wie beispielsweise Thionylchlorid, Phosgen, Phosphorylchlorid, Phosphorpentachlorid, Phosphorylbromid oder Phosphortribromid bei Temperaturen zwischen 50° C und 150° C halogeniert (vgl. EP-OS 259139 und EP-OS 270362).

Die Picolinamin-Derivate der Formel (V) oder deren Säureadditionssalze sind teilweise bekannt und/oder können in Analogie zu bekannten Verfahren hergestellt werden (vgl. EP-OS 259139 und EP-OS 270362).

Die zur Durchführung des erfindungsgemäßen Verfahrens (B) außerdem als Ausgangsstoffe benötigten Verbindungen sind durch die Formel (III) allgemein definiert. In dieser Formel (III) stehen $R^{4-1}$ und M vorzugsweise für diejenigen Reste, die bereits im Zusammenhang mit der Beschreibung der erfindungsgemäßen Stoffe der Formel (I) als bevorzugt für diese Substituenten genannt wurden.

Die Verbindungen der Formel (III) sind allgemein bekannte Verbindungen der organischen Chemie.

Die zur Durchführung des erfindungsgemäßen Verfahrens (C) außerdem als Ausgangsstoffe benötigten Diester-Derivate sind durch die Formel (IV) allgemein definiert. In dieser Formel (IV) stehen $R^{4-2}$ und $R^6$ vorzugsweise für diejenigen Reste, die bereits im Zusammenhang mit der Beschreibung der erfindunsggemäßen Stoffe der Formel (I) als bevorzugt für diese Substituenten genannt wurden.

Die Verbindungen der Formel (IV) sind allgemein bekannte Verbindungen der organischen Chemie.

Die zur Durchführung des erfindungsgemäßen Verfahrens (D-α) außerdem als Ausgangsstoffe benötigten Arylketone sind durch die Formel (VI) allgemein definiert. In dieser Formel (VI) stehen $R^{4-3}$, $R^5$ und Ar für diejenigen Reste, die bereits im Zusammenhang mit der Beschreibung der erfindungsgemäßen Stoffe der Formel (I) als bevorzugt für diese Substituenten genannt wurden.

E steht vorzugsweise für Halogen, insbesondere Chlor, Brom oder Iod, für Dialkylamino, wie beispielsweise Dimethylamino oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy.

Die Verbindungen der Formel (VI) sind allgemein bekannte Verbindungen der organischen Chemie.

Die zur Durchführung des erfindungsgemäßen Verfahrens (D-β) außerdem als Ausgangsstoffe benötigten Acetale sind durch die Formel (VII) allgemein definiert. In dieser Formel (VII) stehen Ar, $R^{4-3}$ und $R^5$ für diejenigen Reste, die bereits im Zusammenhang mit der Beschreibung der erfindungsgemäßen Stoffe der Formel (I) als bevorzugt für diese Substituenten genannt wurden.

$R^7$ steht vorzugsweise für Methyl oder Ethyl.

Die zur Durchführung des erfindungsgemäßen Verfahrens (D-γ) außerdem benötigten 1,3-Dioxo-Derivate sind durch die Formel (VIII) allgemein definiert. In dieser Formel (VIII) stehen Ar, $R^{4-3}$ und $R^5$ für diejenigen Reste, die bereits im Zusammenhang mit der Beschreibung der erfindungsgemäßenStoffe der Formel (I) als bevorzugt für diese Substituenten genannt wurden.

Die Verbindungen der Formeln (VII) und (VIII) sind allgemein bekannte Verbindungen der organischen Chemie.

Die zur Durchführung des erfindungsgemäßen Verfahrens (D-δ) außerdem als Ausgangsstoffe benötigten Diacetale sind durch die Formel (IX) allgemein definiert. In dieser Formel (IX) stehen Ar, $R^{4-3}$ und $R^5$ für diejenigen Reste, die bereits im Zusammenhang mit der Beschreibung der erfindungsgemäßen Stoffe der Formel (I) als bevorzugt für diese Substituenten genannt wurden.

$R^8$ steht vorzugsweise für Methyl oder Ethyl.

Die Verbindungen der Formel (IX) sind allgemein bekannte Verbindungen der organischen Chemie.

Das erfindungsgemäße Verfahren (A) zur Herstellung der neuen Verbindungen der Formel (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören Benzol, Toluol, Xylol, Tetrahydrofuran und Dioxan, Ester wie Essigsäuremethylester und -ethylester, Alkohole, wie Methanol, Ethanol und Isopropanol sowie Wasser. Vorzugsweise verwendet man Wasser, Methanol, Ethanol, Essigsäureethylester und Toluol oder Mischungen dieser Verdünnungsmittel.

Die Reaktionstemperaturen können bei dem erfindungsgemäßen Verfahren (A) in einem größeren Bereich variiert werden. Im allgemeinen arbeitet man bei Temperaturen zwischen 0 °C und +100 °C, vorzugsweise bei Temperaturen zwischen +20 °C und +50 °C.

Das erfindungsgemäße Verfahren (A) wird vorzugsweise unter Verwendung von Katalysatoren durchgeführt. Als solche kommen übliche Hydrierungskatalysatoren infrage, wie beispielsweise Raney-Nickel, elementares Platin oder Palladium, vorzugsweise verwendet man Palladium/Aktivkohle.

Das erfindungsgemäße Verfahren (A) wird in gegenwart von Basen durchgeführt. Als solche kommen beispielsweise Alkalimetallhydroxide wie z.B. Natrium- und Kaliumhydroxid, Erdalkalihydroxide wie z.B. Calciumhydroxid, Alkalicarbonate, -acetate und -alkoholate, wie Natrium- und Kaliumcarbonat, Natrium- und Kaliummethylat bzw. -ethylat, Ammoniak, Amine, wie Diethylamin oder basische Ionenaustauscher infrage.

Zur Durchführung des erfindungsgemäßen Verfahrens (A) arbeitet man im allgemeinen bei einem Wasserstoffdruck von 1 bis 5 Atmosphären, vorzugsweise bei 1 bis 3 Atmosphären. Zur Aufarbeitung wird der Katalysator abfiltriert, die Reaktionslösung mit Wasser versetzt und mit einem organischen Lösungsmittel extrahiert. Die weitere Aufarbeitung erfolgt nach üblichen Methoden.

Zur Durchführung des erfindungsgemäßen Verfahrens (A) setzt man pro Mol an halogeniertem Pyridinylpyrimidin-Derivatder Formel (II) im allgemeinen 1 bis 10 Mol, vorzugsweise 1 bis 5 Mol an Wasserstoff sowie im allgemeinen 0,01 bis 1 Mol, vorzugsweise 0,01 bis 0,1 Mol an Katalysator ein.

Das erfindungsgemäße Verfahren (B) zur Herstellung der neuen Verbindungen der Formel (Ib) wird vorzugsweise unter Verwendung von Verdünnungsmitteln durchgeführt. Als Verdünnungsmittel kommen dabei praktisch alle inerten organischen Lösungsmittel in Frage. Hierzu gehören aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol oder Isopropanol. Vorzugsweise verwedet man Ether wie Diethylether, Dioxan und Tetrahydrofuran sowie Alkohole wie Methanol und Ethanol oder Mischungen dieser Verdünnungsmittel.

Die Reaktionstemperaturen können bei dem erfindungsgemäßen Verfahren (B) in einem größeren Bereich variiert werden. Im allgemeinen arbeitet man bei Temperaturen zwischen 0 °C und +150 °C, vorzugsweise bei Temperaturen zwischen +10 °C und +120 °C.

Das erfindungsgemäße Verfahren (B) wird in Gegenwart von Basen durchgeführt. Vorzugsweise verwendet man Alkalimetallhydroxide wie Natrium- oder Kaliumhydroxid oder organische Basen wie Natriummethanolat, Triethylamin oder Pyridin.

Zur Durchführung des erfindungsgemäßen Verfahrens (B) setzt man pro Mol an halogensubstituiertem Pyridinylpyrimidin-Derivat der Formel (II) im allgemeinen 1 bis 3 Mol, vorzugsweise 1 bis 1,5 Mol an Verbindung der Formel (III) ein.

Zur Durchführung des erfindungsgemäßen Verfahrens (B) wird das Reaktionsgemisch mehrere Stunden bei der erforderlichen Temperatur gerührt. Die Aufarbeitung erfolgt nach üblichen Methoden, eine anschließende Reinigung erfolgt gegebenenfalls durch Chromatographie.

Das erfindungsgemäße Verfahren (C) zur Herstellung der neuen Verbindungen der Formel (Ic) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methyl-ethyl-, Methyl-isopropyl- und

Methyl-isobutyl-keton, Ester wie Essigsäuremethylester und -ethylester, Nitrile wie z. B. Acetonitril und Propionitril, Dimethylsulfoxid und Sulfolan sowie Gemische dieser Verdünnungsmittel.

Die Reaktionstemperaturen können beim ersten Reaktionsschritt des erfindungsgemäßen Verfahren (C) in einem größeren Bereich variiert werden. Im allgemeinen arbeitet man bei Temperaturen zwischen -10 °C und +180 °C, vorzugsweise bei Temperaturen zwischen 0 °C und +150 °C.

Der erste Reaktionsschritt des erfindungsgemäßen Verfahrens (C) wird in Gegenwart von Basen durchgeführt. Vorzugsweise verwendet man Alkalimetallhydride wie beispielsweise Natriumhydrid, Alkyllithium wie n-Butyllithium, Lithiumdialkylamide wie z.B. Lithiumdiisopropylamid (LDA), Alkalimetallalkoholate wie z.B. Natriummethanolat und Natriumethanolat und Metallhydroxide wie z.B. Natriumhydroxid.

Zur Durchführung des ersten Reaktionsschrittes des erfindungsgemäßen Verfahrens (C) setzt man pro Mol an halogensubstituiertem Pyridinylpyrimidin-Derivat der Formel (II) im allgemeinen 0,5 bis 2 Mol, vorzugsweise 1 bis 1,5 Mol an Diester-Derivat der Formel (IV) sowie im allgemeinen 1 bis 3 Mol, vorzugsweise 1 bis 2 Mol an Base ein.

Die Reaktionstemperaturen können beim zweiten Reaktionsschritt des erfindungsgemäßen Verfahrens (C) in einem größeren Bereich variiert werden. Im allgemeinen arbeitet man bei Temperaturen zwischen 0 °C und +150 °C, vorzugsweise bei Temperaturen zwischen +10 °C und +100 °C.

Der zweite Reaktionsschritt des erfindungsgemäßen Verfahrens (C) wird in Gegenwart von Basen durchgeführt. Vorzugsweise verwendet man Alkalimetallhydroxide wie z.B. Natriumhydroxid und Alkalimetallcarbonate wie z.B. Natriumcarbonat.

Zur Durchführung des zweiten Reaktionsschrittes des erfindungsgemäßen Verfahrens (C) setzt man pro Mol an verwendetem halogensubstituierten Pyridinylpyrimidin-Derivat der Formel (II) im allgemeinen 1 bis 6 Mol, vorzugsweise 2,1 bis 5 Mol an Base ein.

Die Reaktionstemperaturen können bei der Decarboxylierungsreaktion des erfindungsgemäßen Verfahrens (C) in einem größeren Bereich variiert werden. Im allgemeinen arbeitet man bei Temperaturen zwischen 0 °C und +200 °C, vorzugsweise bei Temperaturen zwischen +20 °C und +150 °C.

Die Decarboxylierungsreaktion des erfindungsgemäßen Verfahrens (C) wird in Gegenwart von Säuren durchgeführt. Vorzugsweise verwendet man anorganische Säuren wie z.B. Schwefelsäure oder Chlorwasserstoffsäure und organische Säuren wie z.B. Essigsäure.

Zur Durchführung der Decarboxylierungsreaktion des erfindungsgemäßen Verfahrens (C) setzt man pro Mol an verwendetem halogensubstituiertem Pyridinylpyrimidin der Formel (II) im allgemeinen 1 bis 10 Mol, vorzugsweise 2,5 bis 6 Mol an Säure ein.

Die Durchführung des erfindungsgemäßen Verfahrens (C) erfolgt nach üblichen Methoden. Zur Aufarbeitung wird die Reaktionsmischung neutralisiert, konzentriert und extrahiert. Die Reinigung des Produktes erfolgt durch Chromatographie oder Umkristallisation.

Das erfindungsgemäße Verfahren (D-α) zur Herstellung der neuen Verbindungen der Formel (I-d) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise Ether wie Tetrahydrofuran und Dioxan, Amide wie z. B. Dimethylformamid, Dimethylacetamid und Pyridin sowie Alkohole wie Methanol und Ethanol.

Die Reaktionstemperaturen können bei dem erfindungsgemäßen Verfahren (D-α) in einem größeren Bereich variiert werden. Im allgemeinen arbeitet man bei Temperaturen zwischen 0 °C und +200 °C, vorzugsweise bei Temperaturen zwischen +50 °C und +150 °C.

Das erfindungsgemäße Verfahren (D-α) wird in Gegenwart von Basen durchgeführt. Vorzugsweise verwendet man Metallalkoholate wie z.B. Natriummethanolat und Natriumethanolat sowie organische Basen wie z.B. Trimethylamin, Triethylamin und N,N-Diethylanilin.

Die Reaktionsdurchführung und Aufarbeitung erfolgt nach üblichen Methoden durch Einengen unter vermindertem Druck und gegebenenfalls anschließender chromatographischer Reinigung.

Zur Durchführung des erfindungsgemäßen Verfahrens (D-α) setzt man pro Mol an Picolinamidin-Derivat der Formel (V) im allgemeinen 1 bis 3 Mol, vorzugsweise 1 bis 1,5 Mol an Arylketon der Formel (VI) sowie im allgemeinen 0,1 bis 5 Mol, vorzugsweise 0,1 bis 2,5 Mol an Base ein.

Das erfindungsgemäße Verfahren (D-β) zur Herstellung der neuen Verbindungen der Formel (I-d) wird vorzugsweise unter Verwendung von Verdünnungsmitteln durchgeführt. Als Verdünnungsmittel kommen dabei Ether wie Tetrahydrofuran und Dioxan, Amide wie z. B. Dimethylformamid, Dimethylacetamid sowie Pyridin, Wasser oder Mischungen der genannten Lösungsmittel infrage.

Die Reaktionstemperaturen können bei dem erfindungsgemäßen Verfahren (D-β) in einem größeren Bereich variiert werden. Im allgemeinen arbeitet man bei Temperaturen zwischen 0 °C und +200 °C, vorzugsweise bei Temperaturen zwischen +50 °C und +150 °C.

Das erfindungsgemäße Verfahren (D-β) wird in Gegenwart von Basen durchgeführt. Vorzugsweise

verwendet man anorgansiche Basen wie Natrium- oder Kaliumhydroxid oder Kaliumcarbonat oder organische Basen wie Natriummethanolat, Triethylamin oder N,N-Diethylanilin.

Zur Reaktionsaufarbeitung wird von gebildeten anorganischen Salzen abfiltriert. Das Filtrat wird eingeengt und gegebenenfalls chromatographisch oder durch Umkristallisation gereinigt.

Zur Durchführung des erfindungsgemäßen Verfahrens (D-β) setzt man pro Mol an Picolinamidin-Derivat der Formel (V) im allgemeinen 1 bis 3 Mol, vorzugsweise 1 bis 1,5 Mol an Acetal der Formel (VII) sowie im allgemeinen 0,01 bis 3, vorzugsweise 0,1 bis 1 Mol an Base ein.

Das erfindungsgemaße Verfahren (D-γ) wird in Gegenwart von Basen durchgeführt. Vorzugsweise verwendet man Alkalimetallalkoholate wie z.B. Natriummethanolat und Natriumethanolat sowie organsicher Basen wie Triethylamin und N,N-Diethylanilin.

Zur Durchführung des erfindungsgemäßen Verfahrens (D-γ) setzt man pro Mol an Picolinamidin-Derivat der Formel (V) im allgemeinen 1 bis 3 Mol, vorzugsweise 1 bis 1,5 Mol an 1,3-Dioxo-Derivat der Formel (VIII) sowie im allgemeinen 0,1 bis 5 Mol, vorzugsweise 0,1 bis 2,5 Mol an Base ein.

Zur Durchführung des erfindungsgemäßen Verfahrens (D-γ) wird die Reaktionsmischung mehrere Stunden bei der erforderlichen Temperatur gerührt. Die Aufarbeitung und Isolierung des Reaktionsproduktes erfolgt nach üblichen Methoden.

Das erfindungsgemäße Verfahren (D), Variante (δ) wird vorzugsweise in Abwesenheit von Verdünnungsmitteln durchgeführt. Es ist jedoch auch möglich eines der folgenden Verdünnungsmittel zu verwenden: aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid,Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (D) Variante (δ) in einem größeren Bereich variiert werden. Im allgemeinen arbeitet man bei Temperaturen zwischen 0 °C und +200 °C, vorzugsweise bei Temperaturen zwischen +50 °C und +150 °C.

Zur Durchführung des erfindungsgemäßen Verfahrens (D-δ) setzt man pro Mol Picolinamidin-Derivat der Formel (V) im allgemeinen 1 bis 5 Mol, vorzugsweise 1 bis 3 Mol an Diacetal der Formel (IX) ein.

Zur Durchführung des erfindungsgemäßen Verfahrens (D-δ) wird die Reaktionsmischung mehrere Stunden bei der erforderlichen Temperatur gerührt. Die Aufarbeitung erfolgt nach üblichen Methoden durch Einengen unter vermindertem Druck und anschließender
chromatographischer Reinigung.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;

Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Obst- und Gemüsebau, wie beispielsweise gegen den Erreger von Fäule an Tomaten (Phytophthora) sowie zur protektiven Behandlung gegen beispielsweise Apfelschorf (Venturia inaequalis), Bohnengrauschimmel (Botrytis cinerea), gegen Erysiphe graminis an Weizen oder Gerste sowie gegen den Erreger der Braunfleckenkrankheit an Gerste (Cochliobolus sativus) einsetzen.

Weiterhin zeigen einige der erfindungsgemäßen Wirkstoffe gute fungizide Wirkung gegen Septoria nodorum, Pyrenophora teres, gegen Fusarium culmorum, Fusarium nivale sowie gegen Pyricularia oryzae und Pellicularia sasakii. Ferner zeigen einige der erfindungsgemäßen Wirkstoffe auch eine gute und breite in vitro-Wirkung.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffeund Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinenzwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die biologische Wirksamkeit der erfindungsgemäßen Verbindungen wird anhand der Anwendungsbeispiele erläutert.

Herstellungsbeispiele

Beispiel 1

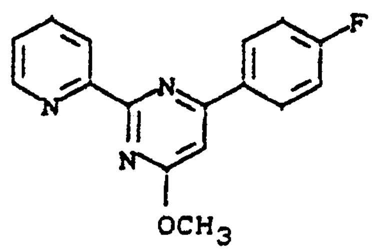

(Verfahren (B))

5,5 g (0,02 Mol) 4-Chlor-2-(2-pyridyl)-6-(4-fluorphenyl)-pyrimidin werden in 50 ml trockenem Methanol gelöst und mit 8,6 g einer 22%-igen Lösung von Natriummethylat in Methanol (entspricht 0,04 Mol) versetzt und 40 Stunden am Rückfluß erhitzt. Nachdem das Lösungsmittel entfernt wurde, wird der erhaltene Rückstand mit Essigester aufgenommen, zweimal mit Wasser gewaschen und über Natriumsulfat getrocknet. Nach Entfernung des Lösungsmittels erhält man 4,3 g (77 % der Theorie) 4-Methoxy-2-(2-pyridyl)-6-(4-fluorphenyl)-pyrimidin vom Schmelzpunkt 97-98 °C.

Beispiel 2

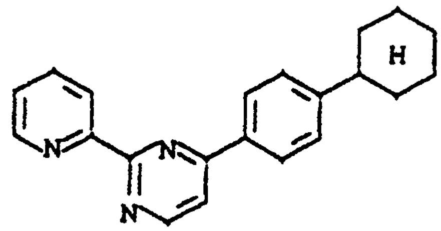

(Verfahren (D-α)

3,2 g (0,02 Mol) 2-Pyridinyl-amidin-Hydrochlorid und 5,1 g (0,02 Mol) 1-(4-Cyclohexyl-phenyl)-3-dimethylamino-propen-on werden in 50 ml trockenem Methanol gelöst und mit 5,2 g einer 22%igen Natriummethylat-Lösung (entspr. 0,024 Mol) versetzt. Man erhitzt 15 Stunden am Rückfluß und neutralisiert die Lösung anschließend mit Essigsäure. Die Lösungsmittel werden unter vermindertem Druck abdestilliert, der Rückstand mit Dichlormethan aufgenommen. Man wäscht die Lösung mit Wasser und trocknet anschließend über Natriumsulfat. Nach Entfernung aller flüchtigen Bestandteile unter stark vermindertem Druck erhält man 5,66 g (94 % der Theorie) 2-(2-Pyridyl)-4-(4-cyclohexyl-phenyl)-pyrimidin als Öl.

$^1$H-NMR *): 7,65; 8,95)

In entsprechender Weise, analog zu den oben beschriebenen Verfahren (A), (B), (C), (D-α), (D-β), (D-γ) und (D-δ) und gemäß den allgemeinen Angaben zur Herstellung erhält man die in Tabelle 2 aufgeführten Pyrimidinylpyrimidin-Derivate der allgemeinen Formel (I):

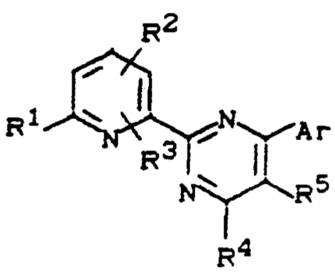

(I)

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Tabelle 2

| Bsp. Nr. | $R^1$ | $R^2$ | $R^3$ | $R^4$ | $R^5$ | Ar | physikal. Daten |
|---|---|---|---|---|---|---|---|
| 3 | H | H | H | H | H | 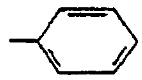 | $n_D^{20}$ :1.6605 |
| 4 | H | H | H | H | H | 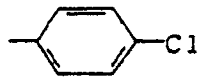 | Fp:109° C |
| 5 | H | H | H | $CF_3$ | H | 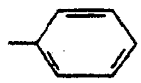 | Fp: 75° C |
| 6 | H | H | H | H | H | 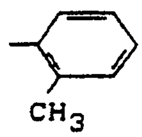 | $n_D^{20}$ :1.6213 |
| 7 | H | H | H | H | H | 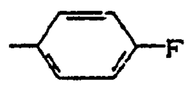 | Fp: 80° C |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 8 | H | H | H | $CH_3$ | H | 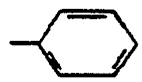 | Öl |
| 9 | H | H | H | H | H | 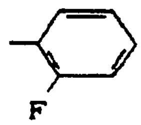 | $n_D^{20}$ :1.6164 |
| 10 | H | H | H | H | H | 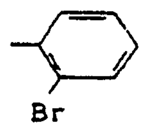 | Fp: 45° C |
| 11 | H | H | H | H | H | 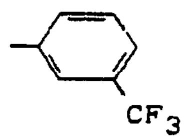 | Fp: 70° C |
| 12 | H | H | H | H | $CH_3$ | 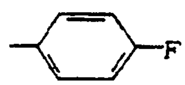 | Fp: 72° C |
| 13 | H | H | H | H | H | 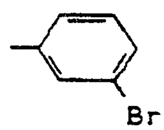 | Öl |
| 14 | H | H | H | H | H | 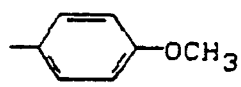 | Öl |
| 15 | H | H | H | H | H | 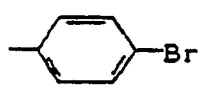 | Öl |
| 16 | H | H | H | H | H | 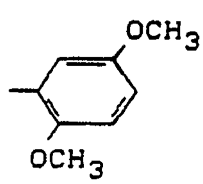 | Öl |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 17 | H | H | H | H | H | 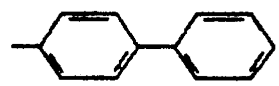 | Fp: 141° C |
| 18 | H | H | H | H | H | 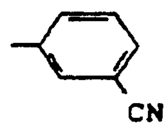 | Fp: 108° C |
| 19 | H | H | H | H | H | 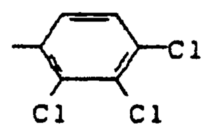 | Fp: 196° C |
| 20 | H | H | H | H | H | 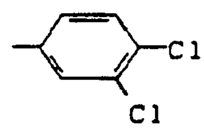 | Fp: 131° C |
| 21 | H | H | H | H | H | 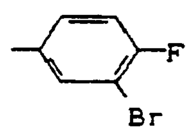 | Öl |
| 22 | H | H | H | H | H | 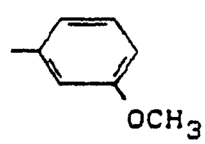 | Öl |
| 23 | H | H | H | H | H | 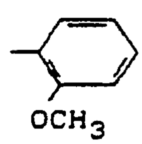 | Öl |
| 24 | H | H | H | H | H | 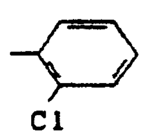 | Fp: 100° C |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 25 | H | H | H | H | H | 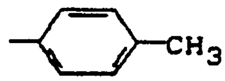 | Fp: 137° C |
| 26 | H | H | H | H | H | 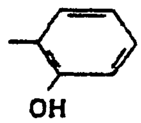 | Fp: 123° C |
| 27 | H | H | H | H | H | 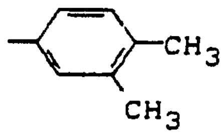 | Öl |
| 28 | H | H | H | H | H | 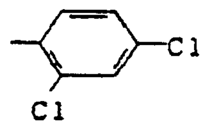 | Fp: 152° C |
| 29 | H | H | H | H | $-C_2H_5$ | 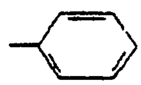 | Öl |
| 30 | H | H | H | H | H | 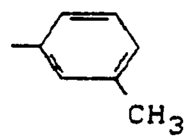 | Öl |
| 31 | H | H | H | H | H | 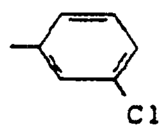 | Öl |
| 32 | H | H | H | H | $CH_3$ | 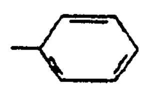 | Öl |
| 33 | H | H | H | H | $CH_3$ | 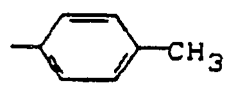 | Öl |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 34 | H | H | H | H | H | 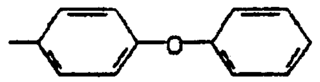 | Öl |
| 35 | H | H | H | H | H | 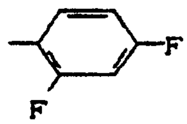 | Fp: 117° C |
| 36 | H | H | H | H | H | 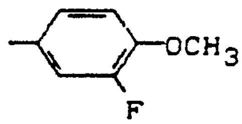 | Fp: 98° C |
| 37 | H | H | H | H | H | 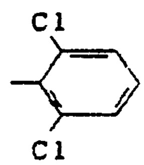 | Öl |
| 38 | H | H | H | $CH_3$ | H | 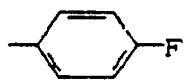 | Fp: 80° C |
| 39 | H | H | H | $CH_3$ | H | 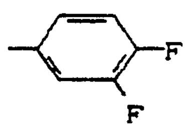 | Fp: 124° C |
| 40 | H | H | H | H | $CH_3$ | 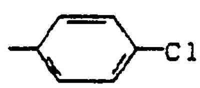 | Fp: 122° C |
| 41 | H | H | H | H | $-C_3H_7$ | 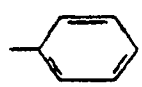 | Fp: 101° C |
| 42 | H | 5-$CF_3$ | H | H | H | 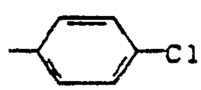 | Fp: 162° C |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 43 | H | 5-$CF_3$ | H | H | H | 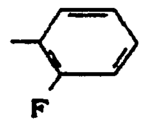 | Fp: 118° C |
| 44 | H | H | H | -$OCH_3$ | H | 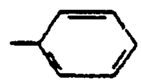 | Fp: 94° C |
| 45 | F | 5-$CF_3$ | H | H | H | 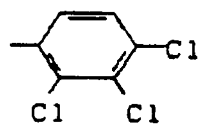 | Fp: 201° C |
| 46 | H | H | H | -$SCH_3$ | H | 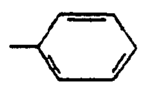 | Fp: 94° C |
| 47 | H | 5-$CF_3$ | H | H | H | 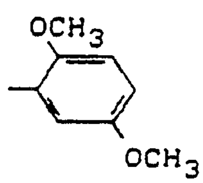 | Fp: 133° C |
| 48 | H | H | H | H | H | 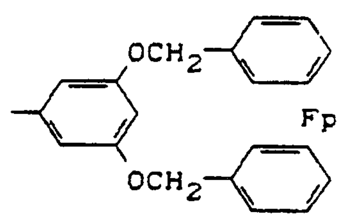 | Fp: 40° C |
| 49 | H | H | H | -$SCH_3$ | H | 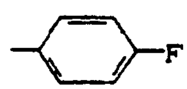 | Fp: 96° C Zers. |
| 50 | H | H | H | -$SC_2H_5$ | H | 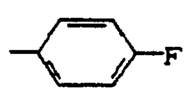 | Öl |
| 51 | H | 5-$CF_3$ | H | H | H | 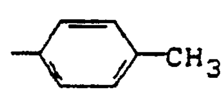 | Fp: 141° C |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 52 | H | H | H | H | H | 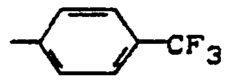 | Fp:135° C |
| 53 | H | H | H | H | H | 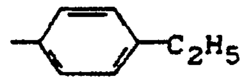 | Fp: 85° C |
| 54 | H | H | H | H | H | 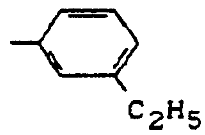 | Öl |
| 55 | H | H | H | H | $CH_3$ | 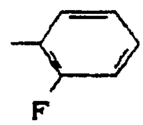 | Fp:124° C |
| 56 | H | H | H | $-SC_2H_5$ | H | 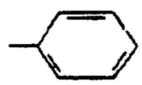 | Öl |
| 57 | H | H | H | $-SC_4H_9$-t | H | 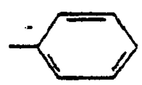 | Fp: 90° C |
| 58 | H | H | H | H | H | 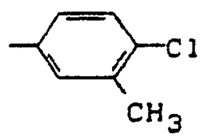 | Öl |
| 59 | H | H | H | H | H | 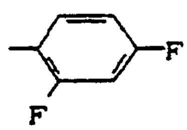 | Öl |
| 60 | H | H | H | $-SC_3H_7$-i | H | 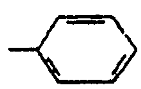 | Öl |

<u>Tabelle 2</u> - Fortsetzung

| Bsp. Nr. | $R^1$ | $R^2$ | $R^3$ | $R^4$ | $R^5$ | Ar | physikal. Daten |
|---|---|---|---|---|---|---|---|
| 61 | H | H | H | $-SC_3H_7$-n | H | 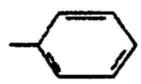 | Fp: 51° C |
| 62 | H | H | H | H | H | 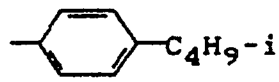 | Öl |
| 63 | H | H | H | H | H | 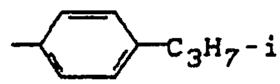 | Fp: 70° C |
| 64 | H | H | H | H | H | 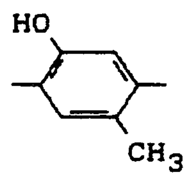 | Fp: 158° C |
| 65 | H | H | H | $SC_4H_9$-n | H | 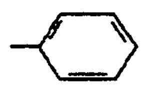 | Fp: 72° C |
| 66 | Br | H | H | H | H | 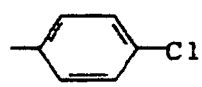 | Fp: 158-160° C |
| 67 | H | H | H | H | H | 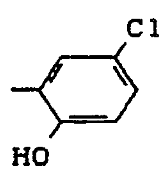 | Fp: 174° C |
| 68 | H | H | H | H | H | 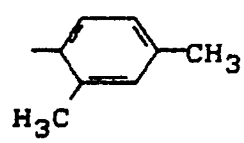 | $^1$H-NMR* δ = (9,0 d, 1H) ppm |
| 69 | Br | H | H | H | H | 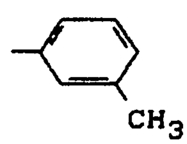 | Fp: 96° C |

Tabelle 2 - Fortsetzung

| Bsp. Nr. | $R^1$ | $R^2$ | $R^3$ | $R^4$ | $R^5$ | Ar | physikal. Daten |
|---|---|---|---|---|---|---|---|
| 70 | H | H | H | H | H | 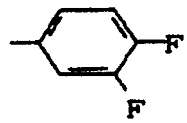 | Fp: 112° C |
| 71 | H | H | H | H | H | 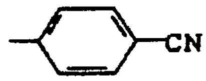 | Fp: 114° C |
| 72 | H | H | H | H | H | 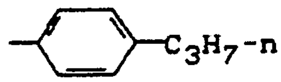 | Fp: 86° C |
| 73 | H | H | H | H | H | 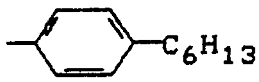 | $^1$H-NMR* δ = 7,7 (d, 1H) ppm |
| 74 | H | H | H | H | H | 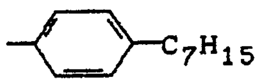 | $^1$H-NMR* δ = 7,7 (d, 1H) ppmm |
| 75 | H | H | H | H | H | 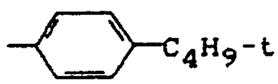 | Fp: 93° C |
| 76 | H | H | H | $OC_2H_5$ | H | 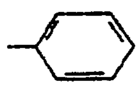 | Fp: 52° C |
| 77 | H | H | H | H | H | 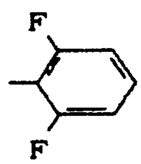 | $^1$H-NMR* δ = 7,5 (m, 1H) ppmm |
| 78 | H | $CH_3$ | H | H | H | 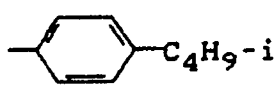 | $^1$H-NMR* δ = 7,65 (d, 1H) ppmm |
| 79 | H | $CH_3$ | H | H | H | 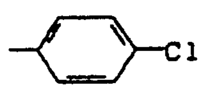 | Fp: 100° C |

* Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm

Herstellung der Ausgangsverbindungen

Beispiel XI-1

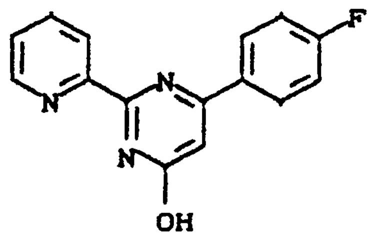

4,2 g (0,18 Mol) Natrium werden in 230 ml trockenem Ethanol gelöst und 22,8 g (0,145 Mol) 2-Pyridinylamidin-Hydrochlorid sowie 32,4 g (0,165 Mol) 4-Fluorbenzoylessigsäuremethylester zugegeben. Die Mischung wird 1 Stunde am Rückfluß erhitzt, anschließend mit konzentrierter Essigsäure neutralisiert und unter vermindertem Druck eingeengt. Dar erhaltene Rückstand wird aus Ethanol umkristallisiert.

Man erhält 18,3 g (46 % der Theorie) 4-Hydroxy-2-(2-pyridyl)-6-(4-fluorphenyl)-pyrimidin vom Schmelzpunkt 197-198 °C.

In entsprechender Weise und gemäß den allgemeinen Angaben zur Herstellung erhält man die in Tabelle 3 aufgeführten Verbindungen der Formel (XI):

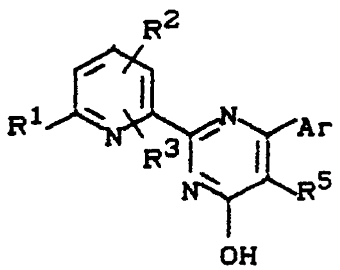

(XI)

Tabelle 3:

| Bsp. Nr. | $R^1$ | $R^2$ | $R^3$ | $R^5$ | Ar | physikal. Konstanten |
|---|---|---|---|---|---|---|
| XI-2 | H | H | H | H | 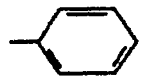 | Fp: 250° C |
| XI-3 | H | H | H | H | 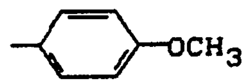 | Fp: 180° C |
| XI-4 | H | H | H | H | 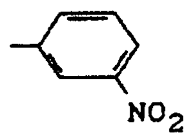 | Fp: 218° C |
| XI-5 | H | H | H | H | 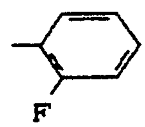 | Fp: 143° C |
| XI-6 | H | H | H | H | 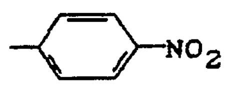 | Fp: >250° C |
| XI-7 | H | 5-$CF_3$ | H | H | 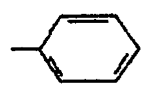 | Fp: 157° C |
| XI-8 | H | 5-$CF_3$ | H | H | 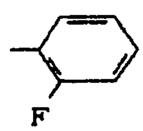 | Fp: 166° C |

Beispiel II-1

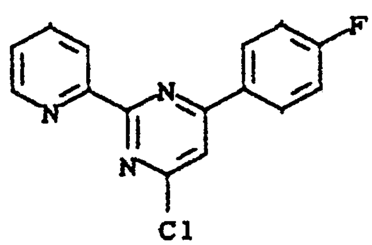

5,3 g (0,02 Mol) 4-Hydroxy-2-(2-pyridyl)-6-(4-fluorphenyl)-pyrimidin und 6,1 g (0,04 Mol) Phosphoroxychlorid werden 2 Stunden am Rückfluß erhitzt. Der erhaltene Rückstand wird abgesaugt und mit

Petrolether gewaschen.

Man erhält 4,9 g (98 % der Theorie) 4-Chlor-2-(2-pyridinyl)-6-(4-fluorphenyl)-pyrimidin vom Schmelzpunkt 156 °C.

In entsprechender Weise und gemäß den allgemeinen Angaben zur Herstellung erhält man die in Tabelle 4 aufgeführten Verbindungen der Formel (II):

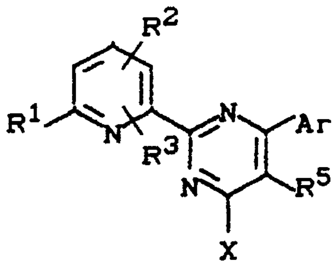

(II)

Tabelle 4:

| Bsp. Nr. | $R^1$ | $R^2$ | $R^3$ | $R^5$ | X | Ar | physikal. Konstanten |
|---|---|---|---|---|---|---|---|
| II-2 | H | H | H | H | Cl | 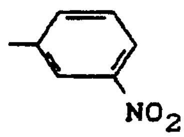 | Fp: 157° C Zers. |
| II-3 | H | H | H | H | Cl | 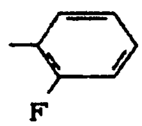 | Fp: 170° C Zers. |
| II-4 | H | H | H | H | Cl | 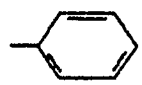 | Fp: 152° C |
| II-5 | H | H | H | H | Cl | 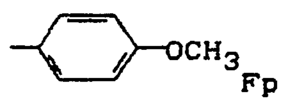 | Fp: 134° C Zers. |
| II-6 | H | H | H | H | Cl | 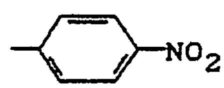 | Fp: 125° C Zers. |

Anwendungsbeispiele:

In den folgenden Anwendungsbeispielen werden die nachstehend aufgeführten Verbindungen als Vergleichssubstanzen eingesetzt:

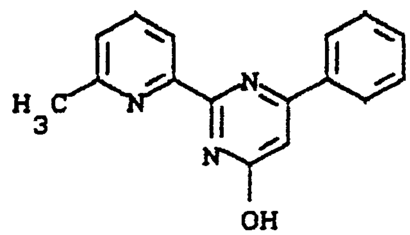

(A)

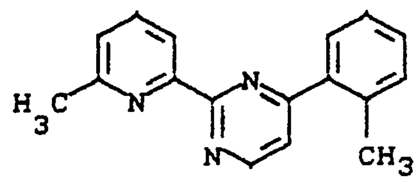

(B)

(bekannt aus EP 0 270 362)

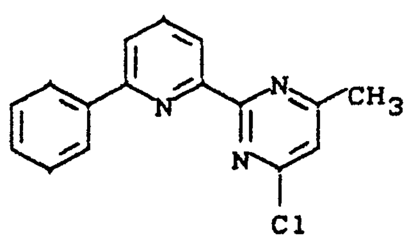

(C)

(bekannt aus EP 0 259 139)

Beispiel A

Phytophthora-Test (Tomate)//kurativ

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 12, 17, 19, 20, 22, 23, 24 und 25.

Beispiel B

Venturia-Test (Apfel) /protektiv

Lösungsmittel: 4,7 Gewichtsteile Aceton

Emulgator: 0,3 Gewichtsteile Alkylarylpolyglkolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 11, 12 und XI-5.

Beispiel C

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen des Herstellungsbeispieles 26.

Beispiel D

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2, 3, 4, 7, 11, 26, 28, 30, 31, II-5 und II-6.

Beispiel E

Erysiphe-Test (Weizen) / protektiv

Lösungsmittel : 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tritici bestäubt.

Die Pflanzen werden in einem Gewächshaus bei einer Temperatur von ca. 20 °C und einer relativen

Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß der Herstellungsbeispiele 3 und II-5.

Beispiel F

Cochliobolus sativus-Test (Gerste)/protektiv

Lösungsmittel: 100 Gewichtsteile Dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 2, 3, 4, 25, 29, 30, 31 und II-1.

## Ansprüche

**1.** Pyridinylpyrimidin-Derivate der allgemeinen Formel (I),

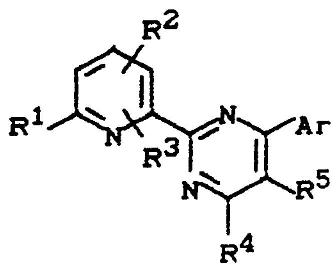

(I)

in welcher

| | |
|---|---|
| $R^1$ | für Wasserstoff, Halogen, Alkoxy, Alkylthio, Halogenalkyl, Amino oder Dialkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist; in welcher weiterhin |
| $R^1$ | für jeweils gegebenenfalls substituiertes Aryloxy, Arylthio, Aralkyloxy oder Aralkylthio steht, |
| $R^2$ und $R^3$ | unabhängig voneinander, gleich oder verschieden jeweils für Wasserstoff, Halogen, Alkyl, Halogenalkyl, Alkoxy, Alkylthio oder Alkoxycarbonyl stehen, oder |
| $R^2$ und $R^3$ | gemeinsam für eine Alkylkette mit 3 bis 6 Kohlenstoffatomen stehen, die über die Ringpositionen 3 und 4 oder 4 und 5 verbunden sind, |
| $R^4$ | für Wasserstoff, Halogen, Alkyl, Halogenalkyl, Hydroxy, Alkoxy, Mercapto, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebe- |

nenfalls ein weiteres Heteroatom enthält und welcher gegebenenfalls durch 1 oder 2 Alkylgruppen substituiert ist; oder

$R^4$ für Alkenyloxy oder Alkinyloxy steht,

$R^5$ für Wasserstoff oder Alkyl steht und

Ar für unsubstituiertes oder substituiertes Aryl steht,

ausgenommen die Verbindungen 4-Phenyl-2-(2-pyridyl)-pyrimidin und 4-Chlor-6-phenyl-2-(2-pyridyl)-pyrimidin.

**2.** Pyridinylpyrimidin-Derivate der Formel (I) gemäß Anspruch 1, bei welchen

$R^1$ für Wasserstoff, Halogen, jeweils geradkettiges oder verzweigtes Alkoxy oder Alkylthio mit jeweils 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, Amino, Alkyl-oder Dialkylamino mit jeweils 1 bis 6 Kohlenstoffatomen in den jeweiligen geradkettigen oder verzweigten Alkylteilen steht, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist; weiterhin für jeweils gegebenenfalls einfach oder mehrfach, gleich oder verschieden substituiertes Phenoxy oder Phenylthio, steht, wobei als Phenylsubstituenten genannt seien: Halogen, Cyano, Nitro, Alkyl, Alkoxy oder Alkylthio mit jeweils 1 oder 2 Kohlenstoffatomen, sowie Halogenalkyl, Halogenalkoxy oder Halogenalkylthio mit jeweils 1 oder 2 Kohlenstoffatomen und jeweils 1 bis 5 Fluor-und/oder Chloratomen oder $R^1$ für jeweils gegebenenfalls einfach oder mehrfach, gleich oder verschieden substituiertes Phenylalkoxy oder Phenylalkylthio mit jeweils 1 bis 4 Kohlenstoffatomen im jeweiligen, geradkettigen oder verzweigten Alkylteil steht, wobei als Substituenten des Phenylteils die oben genannten Phenylsubstituenten infrage kommen,

$R^2$ und $R^3$ unabhängig voneinander, gleich oder verschieden, jeweils für Wasserstoff, Halogen, geradkettiges oder verzweigtes Alkyl mit 1 bis 6 Kohlenstoffatomen, für geradkettiges oder verzweigtes Halogenalkyl mit 1 bis 6 Kohlenstoffatomen und 1 bis 13 gleichen oder verschiedenen Halogenatomen, für jeweils geradkettiges oder verzweigtes Alkoxy oder Alkylthio mit jeweils 1 bis 6 Kohlenstoffatomen, oder für geradkettiges oder verzweigtes Alkoxycarbonyl mit 1 bis 6 Kohlenstoffatomen im Alkoxyteil steht, oder

$R^2$ und $R^3$ gemeinsam für eine Alkylkette mit 3 bis 5 Kohlenstoffatomen stehen, die über die Ringpositionen 3 und 4 oder 4 und 5 verbunden ist,

$R^4$ für Wasserstoff, Halogen, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, Hydroxy, Mercapto, jeweils geradkettiges oder verzweigtes Alkoxy oder Alkylthio mit jeweils 1 bis 6 Kohlenstoffatomen, Amino, Alkyl- oder Dialkylamino mit jeweils 1 bis 6 Kohlenstoffatomen in den jeweiligen, geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel-oder Stickstoffatom enthält und welcher gegebenenfalls durch 1 oder 2 Alkylgruppen mit 1 oder 2 Kohlenstoffatomen substituiert ist, oder für jeweils geradkettiges oder verzweigtes Alkenyloxy oder Alkinyloxy mit jeweils 2 bis 6 Kohlenstoffatomen steht,

$R^5$ für Wasserstoff, geradkettiges oder verzweigtes Alkyl mit 1 bis 6 Kohlenstoffatomen steht und

Ar für gegebenenfalls einfach oder mehrfach, gleich oder verschieden substituiertes Phenyl steht, wobei als Substituenten genannt seien: Halogen, Cyano, Nitro, Hydroxy, Amino, Mercapto, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy, mit jeweils 1 bis 6 Kohlenstoffatomen und jeweils 1 bis 13 gleichen oder verschiedenen Halogenatomen, jeweils

geradkettiges oder verzweigtes Alkenyl oder Alkinyl mit 2 bis 6 Kohlenstoffatomen, jeweils geradkettiges oder verzweigtes Alkenyloxy oder Alkinyloxy mit 2 bis 6 Kohlenstoffatomen, Cycloalkyl mit 3 bis 8 Kohlenstoffatomen, Phenyl, Phenoxy, Phenylthio, Phenylalkylthio oder Phenylalkyloxy mit jeweils 1 bis 4 Kohlenstoffatomen im geradkettigen oder verzweigten Alkylteil, weiterhin seien als Phenylsubstituenten genannt: (Di)alkylamino mit 1 bis 6 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel- oder Stickstoffatom enthält und welcher gegebenenfalls durch 1 oder 2 Alkylgruppen mit 1 oder 2 Kohlenstoffatomen substituiert ist, oder weiterhin Phenylamino, gegebenenfalls 1-oder 2-fach durch geradkettiges oder verzweigtes Alkyl mit 1 bis 6 Kohlenstoffatomen oder Phenyl substituiertes Hydrazino, Alkyl- oder Phenylcarbonylamino mit 1 bis 6 Kohlenstoffatomen im geradkettigen oder verzweigten Alkylteil, Aminocarbonyl, (Di)alkylaminocarbonyl mit 1 bis 6 Kohlenstoffatomen in den jeweiligen geradkettigen oder verzweigten Alkylteilen wobei im Falle von Dialkylaminocarbonyl die beiden Reste gegebenenfalls gemeinsam mit dem Stickstoffatom, an welches sie gebunden sind, einen 5- oder 6-gliedrigen, gesättigten oder ungesättigten Ring bilden, welcher gegebenenfalls ein Sauerstoff-, Schwefel-oder Stickstoffatom enthält und welcher gegebenenfalls durch 1 oder 2 Alkylgruppen mit 1 oder 2 Kohlenstoffatomen substituiert ist sowie geradkettiges oder verzweigtes Alkoxycarbonyl mit 1 bis 6 Kohlenstoffatomen, oder zwei benachbarte Phenylsubstituenten stehen gemeinsam für Alkandiyl mit 1 bis 6 Kohlenstoffatomen, welches gegebenenfalls ein oder mehrere Heteroatome aus der Reihe Sauerstoff, Schwefel und Stickstoff enthält.

3. Pyridinylpyrimidin-Derivate der Formel (I) gemäß Anspruch 1, bei welchen

$R^1$ für Wasserstoff, Fluor, Chlor, Brom, jeweils geradkettiges oder verzweigtes Alkoxy oder Alkylthio mit jeweils 1 bis 4 Kohlenstoffatomen, für geradkettiges oder verzweigtes Halogenalkyl mit 1 bis 4 Kohlenstoffatomen und 1 bis 9 Fluor- und/oder Chloratomen für Amino, Alkyl-oder Dialkylamino mit jeweils 1 bis 4 Kohlenstoffatomen in den jeweiligen geradkettigen oder verzweigten Alkylteilen steht, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel-oder Stickstoffatom enthält und welcher gegebenenfalls durch 1 oder 2 Methylgruppen substituiert ist, weiterhin für jeweils gegebenenfalls ein- oder zweifach, gleich oder verschieden substituiertes Phenoxy oder Phenylthio, wobei als Phenylsubstituenten genannt seien: Fluor, Chlor, Brom, Cyano, Nitro, Methyl, Trifluormethyl, Methoxy, Trifluormethoxy, Methylthio oder Trifluormethylthio, oder $R^1$ für jeweils gegebenenfalls ein- oder zweifach, gleich oder verschieden substituiertes Phenylalkoxy oder Phenylalkylthio mit jeweils 1 oder 2 Kohlenstoffatomen im Alkylteil steht, wobei als Substituenten des Phenylteils die oben genannten Phenylsubstituenten infrage kommen,

$R^2$ für Wasserstoff, Fluor, Chlor, Brom, für geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, für jeweils geradkettiges oder verzweigtes Alkoxy oder Alkylthio mit 1 bis 4 Kohlenstoffatomen, oder für geradkettiges oder verzweigtes Alkoxycarbonyl mit 1 bis 4 Kohlenstoffatomen im Alkoxyteil steht,

$R^3$ für Wasserstoff, Fluor, Chlor, Brom, geradkettiges oder verzweigtes Alkyl mit 1 bis 4 Kohlenstoffatomen steht oder

$R^2$ und $R^3$ gemeinsam für eine Alkylkette mit 3 oder 4 Kohlenstoffatomen stehen, die über die Ringpositionen 3 und 4 oder 4 und 5 verbunden ist,

$R^4$ für Wasserstoff, Fluor, Chlor, Brom, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor-und/oder Chloratomen, Hydroxy, Mercapto, jeweils geradkettiges oder verzweigtes Alkoxy oder Alkylthio mit 1 bis 4 Kohlenstoffatomen, Amino, Alkyl-oder Dialkylamino mit jeweils 1 bis 4 Kohlenstoffatomen in den jeweiligen geradkettigen oder verzweigten Alkylteilen, wobei im Falle von Dialkylamino die beiden Reste gegebenenfalls gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist, oder für jeweils geradkettiges oder verzweigtes Alkenyloxy oder Alkinyloxy mit jeweils 3 bis 4 Kohlenstoffatomen steht,

$R^5$ für Wasserstoff, geradkettiges oder verzweigtes Alkyl mit 1 bis 4 Kohlenstoffatomen steht und

Ar für gegebenenfalls 1- bis 3-fach, gleich oder verschieden substituiertes Phenyl steht, wobei als Substituenten genannt seien: Fluor, Chlor, Brom, Iod, Cyano, Nitro, Hydroxy, Mercapto, Amino, geradkettiges oder verzweigtes Alkyl mit 1 bis 4 Kohlenstoffatomen, geradkettiges oder verzweigtes Halogenalkyl mit 1 bis 4 Kohlenstoffatomen und 1 bis 9 Fluor- und/oder Chloratomen, jeweils geradkettiges oder verzweigtes Alkoxy oder Alkylthio mit jeweils 1 bis 4 Kohlenstoffatomen, jeweils geradkettiges oder verzweigtes Halogenalkoxy oder Halogenalkylthio mit jeweils 1 bis 4 Kohlenstoffatomen und jeweils 1 bis 9 Fluor-und/oder Chloratomen, jeweils geradkettiges oder verzweigtes Alkenyl oder Alkinyl mit jeweils 2 bis 4 Kohlenstoffatomen, jeweils geradkettiges oder verzweigtes Alkenyloxy oder Alkinyloxy mit jeweils 3 bis 4 Kohlenstoffatomen, Cycloalkyl mit 3 bis 6 Kohlenstoffatomen, Phenyl, Phenoxy, Phenylthio, Phenylalkoxy oder Phenylalkylthio mit jeweils 1 oder 2 Kohlenstoffatomen im Alkylteil; weiterhin seien als Phenylsubstituenten genannt:
(Di)alkylamino mit 1 bis 4 Kohlenstoffatomen in den jeweiligen geradkettigen oder verzweigten Alkylteilen, wobei im Falle von Dialkylamino die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel- oder Stickstoffatom enthält und welcher gegebenenfalls durch 1 oder 2 Methylgruppen substituiert ist; weiterhin Phenylamino, gegebenenfalls 1- oder 2-fach durch geradkettiges oder verzweigtes Alkyl mit 1 bis 4 Kohlenstoffatomen oder Phenyl substituiertes Hydrazino, Alkyl- oder Phenylcarbonylamino mit 1 bis 4 Kohlenstoffatomen im geradkettigen oder verzweigten Alkylteil, Aminocarbonyl, (Di-)alkylaminocarbonyl mit 1 bis 4 Kohlenstoffatomen in den jeweiligen geradkettigen oder verzweigten Alkylteilen, wobei im Falle von Dialkylaminocarbonyl die beiden Reste gegebenenfalls, gemeinsam mit dem Stickstoffatom, an welches sie gebunden sind, einen 5- oder 6-gliedrigen, gesättigten Ring bilden, welcher gegebenenfalls ein Sauerstoff-, Schwefel-oder Stickstoffatom enthält und welcher gegebenenfalls durch 1 oder 2 Methylgruppen substituiert ist; sowie geradkettiges oder verzweigtes Alkoxycarbonyl mit 1 bis 4 Kohlenstoffatomen oder zwei benachbarten Phenylsubstituenten stehen gemeinsam für Alkandiyl mit 1 bis 4 Kohlenstoffatomen, welches gegebenenfalls 1 oder 2 Heteroatome aus der Reihe Sauerstoff, Schwefel und Stickstoff enthält.

**4.** Pyridinylpyrimidin-Derivate der Formel (I) gemäß Anspruch 1, bei welchen

$R^1$ für Wasserstoff, Fluor, Chlor, Methoxy, Ethoxy, n-Propoxy oder i-Propoxy, Methylthio, Ethylthio, Trifluormethyl, Trichlormethyl, Difluormethyl oder Chlorfluormethyl, für Methylamino, Dimethylamino, Ethylamino, Diethylamino, Pyrrolidino, Piperidino, Morpholino, Thiomorpholino, n-Methyl-piperazino, 2,6-Dimethylmorpholino, für Phenoxy, Phenylthio, Benzyloxy oder Benzylthio steht,

$R^2$ für Wasserstoff, Fluor, Chlor, Brom, Methyl, Ethyl, n-Propyl oder i-Propyl, für Trifluormethyl, Trichlormethyl, Difluormethyl, Dichlormethyl, für Methoxy oder Ethoxy, für Methylthio, für Methoxy-oder Ethoxycarbonyl steht,

$R^3$ für Wasserstoff, Fluor, Chlor, Brom, Methyl, Ethyl, n-Propyl, i-Propyl oder t-Butyl steht,

$R^4$ für Wasserstoff, Fluor, Chlor, Brom, Methyl, Trifluormethyl, Trichlormethyl, Hydroxy, Mercapto, Methoxy, Ethoxy, n-Propoxy, i-Propoxy, Methylthio, Ethylthio, n-Propylthio oder i-Propylthio, n-Butylthio, i-Butylthio, s-Butylthio oder t-Butylthio, für Amino, Methylamino, Ethylamino, Dimethylamino, Diethylamino, Pyrrolidino, Piperidino, Morpholino, Thiomorpholino, N-Methylpiperazino oder 2,6-Dimethylmorpholino, für 2-Propenyloxy oder 2-Propinyloxy steht,

$R^5$ für Wasserstoff, Methyl, Ethyl, n-Propyl, i-Propyl oder n-Butyl steht und

Ar für gegebenenfalls 1- bis 3-fach, gleich oder verschieden substituiertes Phenyl steht, wobei

als Substituenten genannt seien: Fluor, Chlor, Brom, Iod, Cyano, Nitro, Hydroxy, Amino, Mercapto, Methyl, Ethyl, n-Propyl oder i-Propyl, n-Butyl, s-Butyl, i-Butyl oder t-Butyl, Trifluormethyl, Trichlormethyl, Difluormethyl, Methoxy, Ethoxy, Methylthio, Ethylthio, Trifluormethoxy, Difluormethoxy, Trifluormethylthio, Chlordifluormethylthio, Vinyl, 2-Propenyl, 2-Propinyl, 2-Propenyloxy, 2-Propinyloxy, Cyclopropyl, Cyclopentyl, Cyclohexyl, Phenyl, Phenoxy, Phenylthio, Phenylmethoxy oder Phenylmethylthio, weiterhin seien als Phenylsubstituenten genannt: Methylamino, Dimethylamino, Ethylamino, Diethylamino, Pyrrolidino, Piperidino, Morpholino, Thiomorpholino, N-Methylpiperazino, 2,6-Dimethylmorpholino, Phenylamino, Hydrazino, 2-Methylhydrazo, 2,2-Dimethylhydrazo, 2-Phenylhydrazo, Methylcarbonylamino, Phenylcarbonylamino, Aminocarbonyl, Methylaminocarbonyl, Dimethylaminocarbonyl, Methoxycarbonyl oder Ethoxycarbonyl.

5. Verfahren zur Herstellung von Pyridinylpyrimidin-Derivate der Formel (I)

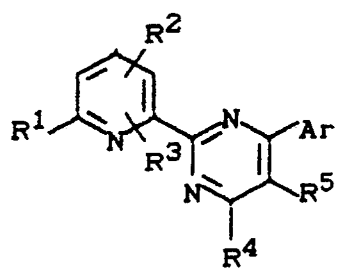

(I)

in welcher

$R^1$ für Wasserstoff, Halogen, Alkoxy, Alkylthio, Halogenalkyl, Amino oder Dialkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist;
in welcher weiterhin

$R^1$ für jeweils gegebenenfalls substituiertes Aryloxy, Arylthio, Aralkyloxy oder Aralkylthio steht,

$R^2$ und $R^3$ unabhängig voneinander, gleich oder verschieden jeweils für Wasserstoff, Halogen, Alkyl, Halogenalkyl, Alkoxy, Alkylthio oder Alkoxycarbonyl stehen,
oder

$R^2$ und $R^3$ gemeinsam für eine Alkylkette mit 3 bis 6 Kohlenstoffatomen stehen, die über die Ringpositionen 3 und 4 oder 4 und 5 verbunden sind,

$R^4$ für Wasserstoff, Halogen, Alkyl, Halogenalkyl, Hydroxy, Alkoxy, Mercapto, Alkylthio, Amino, (Di)alkylamino, steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist, oder

$R^4$ für Alkenyloxy oder Alkinyloxy steht,

$R^5$ für Wasserstoff oder Alkyl steht und

Ar für unsubstituiertes oder substituiertes Aryl steht,

dadurch gekennzeichnet, daß man

A) Pyridinylpyrimidin-Derivate der Formel (Ia)

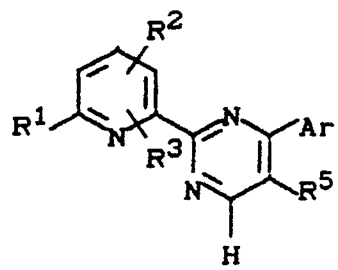

(Ia)

in welcher
$R^1$, $R^2$, $R^3$, $R^5$ und Ar die oben angegebene Bedeutung haben,
erhält,
wenn man halogensubstituierte Pyridinylpyrimidin-Derivate der Formel (II)

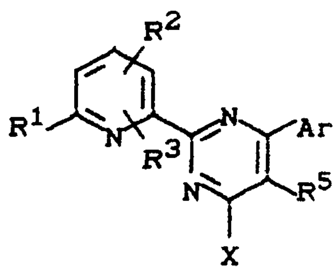

(II)

in welcher
$R^1$, $R^2$, $R^3$, $R^5$ und Ar die oben angegebene Bedeutung haben, und
X für Halogen steht,
gegebenenfalls in Gegenwart eines Lösungsmittels, gegebenenfalls in Gegenwart einer Base, in Gegenwart eines Katalysators und unter Wasserstoffdruck enthalogeniert,
oder
B) Pyridinylpyrimidin-Derivate der Formel (Ib)

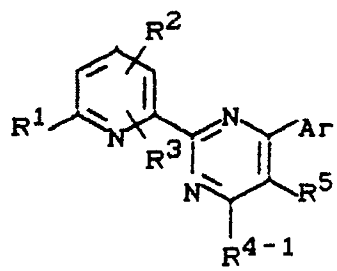

(Ib)

in welcher
$R^1$, $R^2$, $R^3$, $R^5$ und Ar die oben angegebene Bedeutung haben, und
$R^{4-1}$ für Mercapto, Alkoxy, Alkylthio, Amino oder (Di)alkylamino steht, wobei im Falle von Dialkylamino die beiden Reste gegebenenfalls gemeinsam mit dem Stickstoffatom, an welches sie gebunden sind, einen 5- bis 7-gliedrigen, gesättigten oder ungesättigten Ring bilden, welcher gegebenenfalls ein weiteres Heteroatom enthält und welcher gegebenenfalls durch 1 oder 2 Alkylgruppen substituiert ist oder für Alkenyloxy oder Alkinyloxy steht,
erhält,
wenn man halogensubstituierte Pyridinylpyrimidin-Derivate der Formel (II)

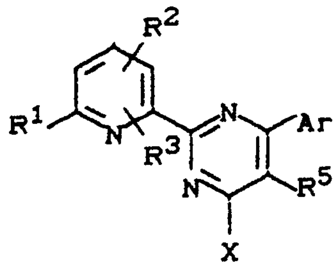

(II)

in welcher

$R^1$, $R^2$, $R^3$, $R^5$ und Ar die oben angegebene Bedeutung haben und

X für Halogen steht,

mit Verbindungen der Formel (III)

$$R^{4-1}\text{-}M \qquad (III)$$

in welcher

$R^{4-1}$ die oben angegebene Bedeutung hat und

M für Wasserstoff oder für ein Alkalimetallkation steht,

gegebenenfalls in Gegenwart eines Verdünnungsmittels und gegebenenfalls in Gegenwart einer Base umsetzt,

oder

C) Pyridinylpyrimidin-Derivate der Formel (Ic)

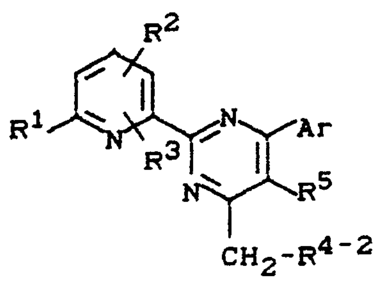

(Ic)

in welcher

$R^1$, $R^2$, $R^3$, $R^5$ und Ar die oben angegebene Bedeutung haben und

$R^{4-2}$ für Wasserstoff oder Alkyl steht,

erhält,

wenn man halogensubstituierte Pyridinylpyrimidin-Derivate der Formel (II)

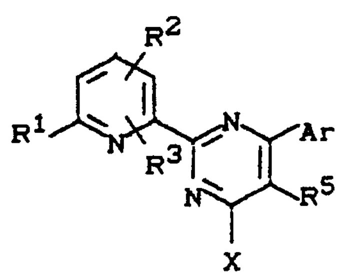

(II)

in welcher

$R^1$, $R^2$, $R^3$, $R^5$ und Ar die oben angegebene Bedeutung haben und

X für Halogen steht,
mit Diester-Derivaten der Formel (IV)

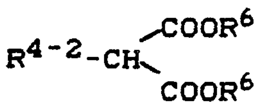

(IV)

in welcher
$R^{4-2}$ die oben angegebene Bedeutung hat und
$R^6$ für Methyl oder Ethyl steht,
gegebenenfalls in Gegenwart eines Verdünnungsmittels und gegebenenfalls in Gegenwart einer Base umsetzt und die so erhaltenen substituierten Pyridinylpyrimidine gegebenenfalls in Gegenwart eines Verdünnungsmittels und in Gegenwart einer Base hydrolysiert und anschließend in Gegenwart einer Säure decarboxyliert;
oder
D) die Pyridinylpyrimidin-Derivate der Formel (Id)

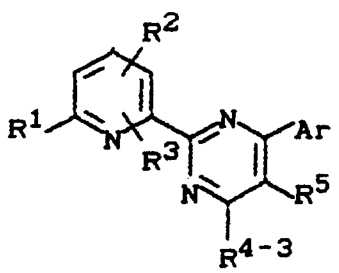

(Id)

in welcher
$R^1$, $R^2$, $R^3$, $R^5$ und Ar die oben angegebene Bedeutung haben und
$R^{4-3}$ für Wasserstoff, Alkyl oder Halogenalkyl steht,
erhält,
wenn man Picolinamidin-Derivate der Formel (V)

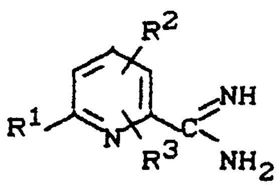

(V)

in welcher
$R^1$, $R^2$ und $R^3$ die oben angegebene Bedeutung haben, oder deren Säureadditionssalze
α) mit Arylketonen der Formel (VI)

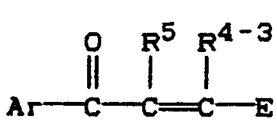

(VI)

in welcher
$R^5$ und Ar die oben angegebene Bedeutung haben,

$R^{4-3}$ für Wasserstoff, Alkyl oder Halogenalkyl steht und

E für eine Abgangsgruppe steht,

gegebenenfalls in Gegenwart eines Verdünnungsmittels und gegebenenfalls in Gegenwart einer Base cyclisiert,

oder

β) mit Acetalen der Formel (VII)

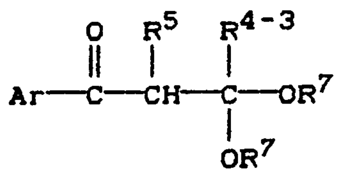

(VII)

in welcher

$R^5$ und Ar die oben angegebene Bedeutung haben,

$R^{4-3}$ für Wasserstoff, Alkyl oder Halogenalkyl steht und

$R^7$ für Alkyl mit 1 bis 4 Kohlenstoffatomen steht,

gegebenenfalls in Gegenwart eines Verdünnungsmittels und gegebenenfalls in Gegenwart einer Base umsetzt,

ν) mit 1,3-Dioxo-Derivaten der Formel (VIII)

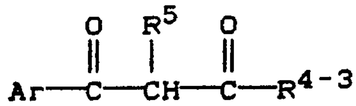

(VIII)

in welcher

$R^5$ und Ar die oben angegebene Bedeutung haben und

$R^{4-3}$ für Wasserstoff, Alkyl oder Halogenalkyl steht,

gegebenenfalls in Gegenwart eines Verdünnungsmittels und in Gegenwart einer Base umsetzt,

oder

δ) mit Diacetalen der Formel (IX)

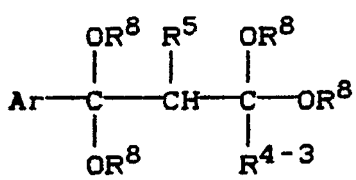

(IX)

in welcher

Ar und $R^5$ die oben angegebene Bedeutung haben,

$R^{4-3}$ für Wasserstoff, Alkyl oder Halogenalkyl steht und

$R^8$ für Alkyl mit 1 bis 4 Kohlenstoffatomen steht,

gegebenenfalls in Gegenwart eines Verdünnungsmittels umsetzt.

**6.** Schädlingsbekämpfungsmittel, gekennzeichnet durch einen Gehalt an mindestens einem Pyridinylpyrimidin-Derivat der Formel (I) nach den Ansprüchen 1 bis 5.

**7.** Verwendung von Pyridinylpyrimidin-Derivaten der Formel (I) nach den Ansprüchen 1 bis 5 zur Bekämpfung von Schädlingen.

8. Verfahren zur Bekämpfung von Schädlingen, dadurch gekennzeichnet, daß man Pyridinylpyrimidin-Derivate der Formel (I) nach den Ansprüchen 1 bis 5 auf Schädlinge und/oder ihren Lebensraum einwirken läßt.

9. Verfahren zur Herstellung von Schädlingsbekämpfungsmitteln, dadurch gekennzeichnet, daß man Pyridinylpyrimidin-Derivate der Formel (I) nach den Ansprüchen 1 bis 5 mit Streckmitteln und/oder oberflächenaktiven Mitteln vermischt.

10. Pyridinylpyrimidin-Derivate der Formel (II)

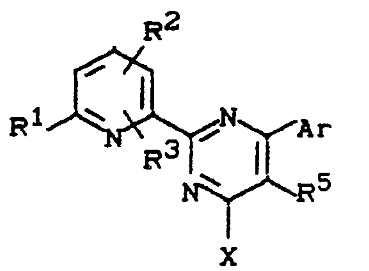

(II)

in welcher
$R^1$, $R^2$, $R^3$, $R^5$ und Ar die in Anspruch 1 angegebene Bedeutung haben, und
X für Halogen steht.

11. Pyridinylpyrimidin-Derivate der Formel (XI)

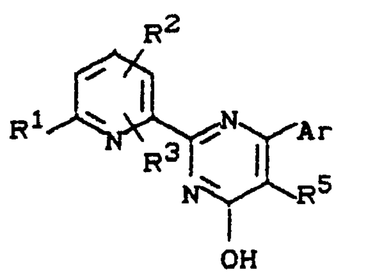

(XI)

in welcher
$R^1$, $R^2$, $R^3$, $R^5$ und Ar die in Anspruch 1 angegebene Bedeutung haben.